# EUROPEAN PATENT APPLICATION

(11) **EP 2 609 953 A1**
(43) Date of publication of application: **03.07.2013**
(21) Application number: 12008262.3
(22) Date of filing: 11.12.2012
(51) Int. Cl.: A61M 5/32, A61M 5/158

(54) **Safety apparatus for medical needles**

(30) Priority: 28.12.2011 JP 2011080372
(71) Applicant: Kawasumi Laboratories, Inc., Oita 876-0121 (JP)
(72) Inventor: Morita, Masayuki, Bungo-Ono-shi, Oita, 8797153 (JP); Watanabe, Masatoshi, Saiki-shi, Oita, 870121 (JP)
(74) Representative: Hering, Hartmut

(57) **Abstract**

Provided is a safety apparatus for medical needles which is free from accidental exposure of an acuate top end contaminated with a blood, etc. from a lower portion thereof. The safety apparatus includes a shield member (3) having a shield hub (35), a shield member connection hub (40H) and leg portions (31,32), the leg portions (31,32) being capable of extending from the first folded position to the second extended position, a needle (21) being to be inserted in the space (40S) of the above shield member connection hub (40H) when the leg portions (31,32) are in the first folded position, the wall (43DW) of the above slide-member (42) being to be pulled into the shield member connection hub (40H) when the leg portions (31,32) are extended up to the second extended position, whereby the apparatus is so formed that it can prevent the distal end (23) of the needle (21) from being exposed to an outside.

## Description

### [Technical Field]

This invention relates to a safety apparatus for medical needles, and specifically it relates to a medical needle safety apparatus having a shield member (protective member) for preventing the dangerous exposure of medical needle that is once used, leading to an accidental needle sticking.
This invention has been made for performing protection (shielding) from a bent needle (having a shape with a bent at approximately 90 degrees with regard to a needle hub) that constitutes, for example, a port access infusion set (called "PAIS"), and in particular it relates to new and useful improvements in a medical needle safety apparatus or new and useful improvements in the form of a shield member (protective member) for bent needles.
The above "port" refers to a member that is implanted in a patient's body, and it is a member that constitutes a system for injecting a liquid medicine such as anticancer drugs through this member from a medical needle.

### [Background Art]

As prior art of the above safety apparatus for medical needles, Patent Document 1 typically describes the following safety apparatus for medical needles. That is, it describes a medical needle safety apparatus comprising: a shield being extensible from a retracted position to an extended position to enclose a distal end of a needle, said needle including a proximal end mounted to a hub and said shield comprising: an outer bearing having a sidewall defining a first interior space about a longitudinal axis; an inner bearing having a sidewall defining a second interior space about said longitudinal axis, wherein said needle is disposed in said second interior space and is movable along said longitudinal axis; said inner bearing disposed in said first interior space and moveable therein along said longitudinal axis; a wedging portion movable with said inner bearing for wedging against said needle to secure the distal end of said needle within the second interior space in the extended position; and a tether having a proximal end connected to said hub and a distal end connected to said inner bearing for preventing separation of the shield from the needle in the extended position.

### [Prior Art Documents]

### [Patent Document]

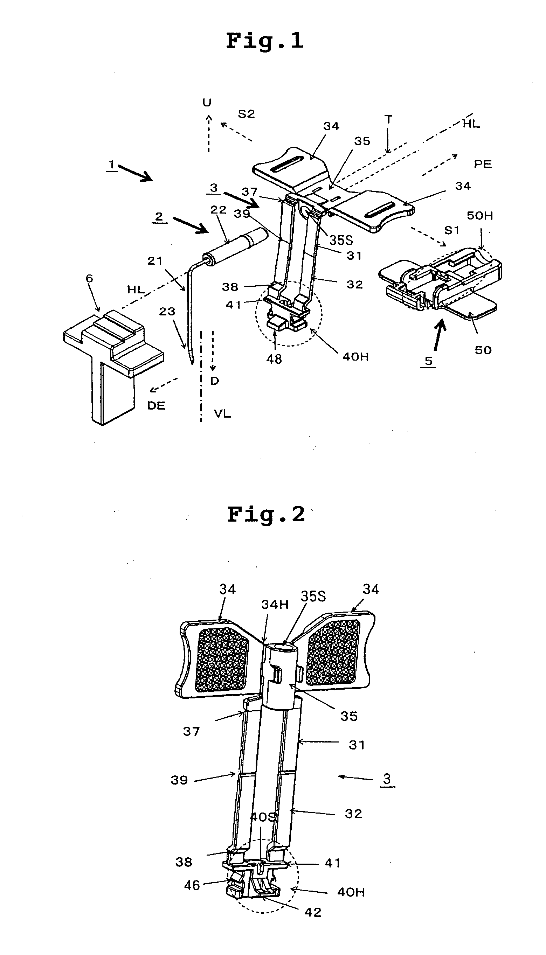

Japanese Translation Version No. 2007-511285 of PCT application (claims, Figures. 1-7 and Figures 16-28; Patent family US Patent 7351230)

### [Summary of the Invention]

### [Problems to be Solved by the Invention]

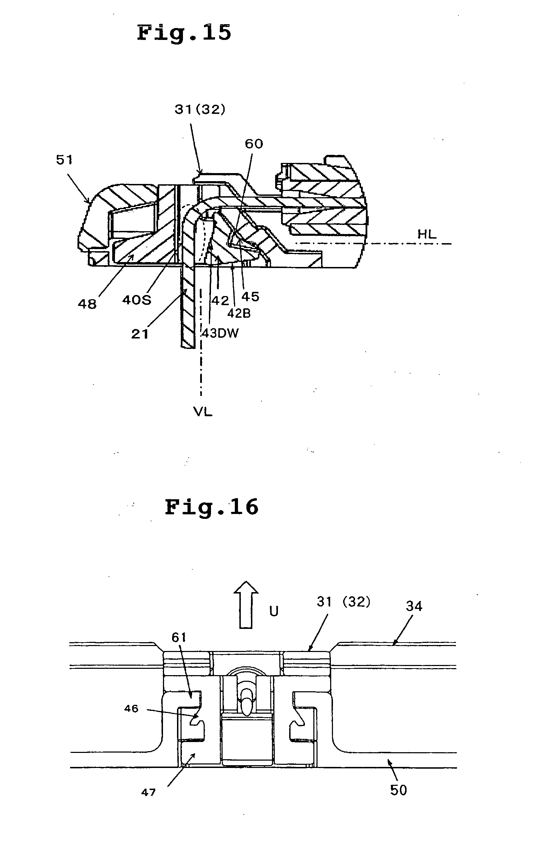

In a medical needle safety apparatus 10 shown in particular in Figures 1 to 7 and a medical needle safety apparatus 110 shown in Figures 16-28 described in detail in above Patent Document 1, that acuate top (distal end) 14, 114 of a medical needle 12, 112 which is taken out of a patient's body and to which patient' blood and fluid are adhering is enclosed with an interior wall surface of a wedging portion 52, 152, while there is a problem that a downward portion is exposed with the acuate top 14, 114 of the medical needle 12, 112 not completely enclosed.
There is hence a concern that the acuate top 14, 114 of the above medical needle 12, 112 contaminated with a patient's blood, etc., containing pathogenic virus, etc., is accidentally exposed from its downward portion to sting a healthcare worker or medical worker (a medical doctor, a nurse, etc.) and may cause an infection case such as a viral infection, etc.
Further, when a patient's blood containing pathogenic germs adheres to the above acuate top 14, 114, contaminant blood may accidentally sputter, in an unhygienic manner.

In a shield assembly 22, 122 of a safety apparatus 10 shown in Figures 1 to 7 and a safety apparatus 110 shown in Figures 16 to 28 in Patent Document 1, further, hinged portions 44A, 44B, 144A, 144B, an inner bearing 24, 124, an outer bearing 26, 126 and a wedging portion 52, 152 are formed from separate parts, respectively, and they are assembled when the safety apparatus is produced. There is hence caused a problem with the prior apparatus that the number of parts is increased, which leads to more labor required for assembling and an increase in cost.

### [Means to Solve the Problems]

With regard to a medical needle in prior art typified by Patent Document 1, it is an object of this invention to provide a safety apparatus for medical needles, in which the top end of a needle that is once used is reliably contained or confined in a safety apparatus or fixed within an apparatus, the top end that is acuate is in no case accidentally exposed from a downward portion of the apparatus and no adhering blood that may fly apart.
It is another object of this invention to provide a safety apparatus for medical needles, the number of parts of which is small, which can be easily assembled and which can be economically produced.
This invention has been made for achieving the above objects, and according to this invention, the following novel safety apparatus for medical needles are provided.
This invention consists of two inventions (first embodiment and second embodiment) featured by a common technical thought as following [1] to [15].

[1] A safety apparatus (1,1') for medical needles, which includes a needle member (2), a shield member (3,3') and a fixing member (5,5'), said needle member (2) having a needle (21) having a distal end (23), said needle (21) is fixed to a needle hub (22) on a proximal end PE side,
   said shield member (3,3') having a shield hub (35), a shield member connection hub (40H,40H') and leg portions (31,32),
   said fixing member (5,5') having a fixing member connection hub (50H,50H'),
   the shield member connection hub (40H,40H') of said shield member (3,B) having a first slide member (42) or a second slide member (42'),
   said shield member connection hub (40H,40H') having formed a space (40S) capable of containing or accommodating the distal end (23) of said needle (21) and a wall (43DW) of said first slide member (42), or walls (42B',45') of said second slide member (42')
   said shield member connection hub (40H,40H') having an opening portion (400, 400') through which the needle (21) can pass toward a downward portion D,
   said leg portions (31,32) being extendable from a first folded position to a second extended position,
   said wall (43DW) of the first slide member (42) shifting together with said leg portions (31, 32) up to a position where it can block the cross section of said space (40S) when said leg portions (31,32) extends from a first folded position to a second extended position, or said walls (42B',45') of said second slide member (42') shifting together with said leg portions (31,32) up to a position where they can block the cross section of the opening portion (40O') of said connection hub (40H') when said leg portions (31,32) extend from the first folded position to the second extended position,
   said needle (21) being inserted into the space (40S) of said connection hub (40H, 40H') when said leg portions (31,32) are in the first folded position, the wall (43DW) of said first slide member (42) or said walls (42B',45') of said second slide member (42') being pulled into the space (40S) of said shield member connection hub (40H, 40H'), when said leg portions (31,32) are in the second extended position,
   whereby the wall (43DW) of said first slide member (42) blocks the cross section of the space (40S) of said shield member connection hub (40H) to prevent the distal end (23) of said needle from moving to a downward portion D lower than the wall (43DW) of said slide member (42), or
   whereby the wall (42B') of said second slide member (42') blocks the cross section of the opening portion (40O') of said shield member connection hub (40H') to prevent the distal end (23) of said needle (21) from moving to the downward portion D lower than the wall (42B') of said slide member (42'), and the apparatus being so formed as to prevent the distal end (23) of said needle (21) from being exposed to an outside.

[2] The safety apparatus (1,1') for medical needles as recited in the above [1], wherein the shield member connection hub (40H, 40H') of said shield member (3,3') has said first slide member (42), or said second slide member (42'),each formed on the proximal end PE side,
   has a shield member first latching portion (48) formed on the distal end DE side, and has a shield member second latching portion (46) and a shield member third latching portion (47) formed on each of a first side portion S1 side and a second side portion S2 side, and
   said shield member second latching portion (46) is formed in an upper portion U above said shield member third latching portion (47).

[3] The safety apparatus (1,1') for medical needles as recited in the above [1] or [2], wherein the fixing member connection hub (50H,50H') of said fixing member (5,5') has a plurality of walls (52,57,63) and a cover (51,51') having a wall (51W),
   the space of said fixing member connection hub (50H,50H') is sectioned into a first space (53) and a second space (59),
   by said plurality of walls (52,57,63) and said wall (51W) of said cover (51,51'),
   and said first space (53) can contain or accommodate the shield member connection hub (40H, 40H') of said shield member (3,3').

[4] 4. The safety apparatus(1,1') for medical needles in any one of the above [1] to [3], wherein the plurality of walls (52,57,63) of the fixing member connection hub (50H,50H') of said fixing member (5,5') has,
   a pair of first side walls (52) extending in the longitudinal direction of a horizontal axial line HL,
   a second side wall (63) formed in the direction from the first side portion S1 to the second side portion S2 on the proximal end PE side of the fixing member connection hub (50H),
   an intermediate wall (57) formed somewhere on said pair of first side walls (52) in the direction from the first side portion S1 to the second side portion S2, and
   a slide wall (60) formed on the proximal end side of said intermediate wall (57).

[5] The safety apparatus (1) for medical needles as recited in any one of the above [1] to [4], wherein the plurality of walls (52,57,63) of fixing member connection hub (50H) of said fixing member (5) has,
   a pair of first side walls (52) extending in the longitudinal direction of a horizontal axial line HL,
   a second side wall (63) formed in the direction from the first side portion S1 to the second side portion S2 on the proximal end PE side of the fixing member connection hub (50H),
   an intermediate wall (57) formed somewhere on said pair of first side walls (52) in the direction from the first side portion S1 to the second side portion S2, and
   a slide wall (60) formed on the proximal end side of said intermediate wall (57), and
   a third latching portion (58) is formed on the distal end side of said intermediate wall (57),and
   when the shield member connection hub (40H) of said shield member (3) and the connection hub (50H) of said fixing member (5) are connected to each other,
   the fixing member third latching portion (58) of fixing member connection hub (50H) of said fixing member (5) is latched and kept fixed by the groove (43) of first slide member (42) of said shield member (3), and
   the slide wall (45) of said first slide member (42) is brought into contact with the slide wall (60) of said intermediate wall (57), and
   when said leg portions (31,32) extend from the first folded position to the second extended position, the first slide member (42) of the said shield member connection hub (40H) is therewith pulled into the space (40S) of said connection hub (40H), and
   the distal end side wall (43DW) of said first slide member (42) shifts up to a position where it can block the cross section of said space (40S).

[6] The safety apparatus (1) for medical needles as recited in any one of the above [1] to [5], wherein the first slide member (42) of shield member connection hub (40H) of said shield member (3) has
   a distal end side wall (43DW) and a downward portion wall (42B),
   a slide wall (45) is formed on the first side portion S1 side and the second side portion S2 side, and
   a groove (43) is formed in said slide wall (45).

[7] The safety apparatus (1,1') for medical needles as recited in any one of claims 1 to 6, wherein, when said leg portions (31,32) are in the first folded position,
   the second latching portion (46) of shield member connection hub (40H, 40H') of said shield member (3,3') is in a downward position of first upper wall (61) of fixing member connection hub (50H,50H') of said fixing member (5,5'),
   and when said leg portions (31,32) extend from the first folded position to the second extended position,
   the shield member second latching portion (46) of said shield member connection hub (40H, 40H') is latched and kept fixed by an upper portion of the first upper wall (61), and the shield member third latching portion (47) of said shield member connection hub (40H, 40H') is latched and kept fixed by a downward portion of the first upper wall (61),
   so that said shield member connection hub (40H, 40H') can be prevented from coming off from the fixing member connection hub (50H,50H') of said fixing member (5,5').

[8] The safety apparatus (1) for medical needles as recited in any one of the above [4] to [7], wherein a groove (43) is formed in the slide wall (45) of shield member connection hub (40H) of said shield member (3) and the fixing member third latching portion (58) of intermediate wall (57) of fixing member connection hub (50H) of said fixing member (5) is formed in the form of a rib for intrudingly latching and keeping it fixed in said groove (43), or
   wherein a latching portion in the form of a rib is formed in the slide wall (45) of shield member connection hub (40H) of said shield member (3), and a groove is formed in intermediate wall (57) of fixing member connection hub (50H) of said fixing member (5) for lathing said latching portion in the form of a rib and keeping it fixed.

[9] The safety apparatus (1') for medical needles as recited in any one of the above [4] to [8], wherein the second slide member (42') of shield member connection hub (40H') of said shield member (3') has a slide wall (45') and a downward portion wall (42B'), and
   wherein, when the shield member connection hub (40H') of said shield member (3') and the fixing member connection hub (50H') of said fixing member (5') are connected to each other,
   the slide wall (45') of said second slide member (42') is brought into contact with the slide wall (60) of said intermediate wall (57), and
   when said leg portions (31,32) extend from the first folded position to the second extended position, the second slide member (42') of said shield member connection hub (40H') is cooperatively therewith pulled into the space (40S') of said shield member connection hub (40H'), and
   the downward portion wall (42B') of said second slide member (42') can shift up to a position where it can block the cross section of said opening portion (40O')

[10] The safety apparatus (1') for medical needles as recited in any one of the above [3] to [9], wherein said cover (51') has a novel fixing member second latching portion (56') in an upper portion U, and
   when the shield member connection hub (40H') of said shield member (3') and the fixing member connection hub (50H') of said fixing member (5') are connected to each other,
   said novel fixing member second latching portion (56') presses the shield member connection hub (40H') of said shield member (3') from the direction of an upper portion U to keep the cover (51') that is once closed from opening again.

[11] The safety apparatus (1,1') for medical needles as recited in any one of the above [1] to [10], wherein said shield hub (35), said leg portions (31,32) and said shield member connection hub (40H,40H') of said shield member (3,3') are an integrated product formed from the same material by integral molding.

[12] The safety apparatus (1,1') for medical needles as recited in any one of the above [1] to [11], wherein said fixing member (5,5') has a needle guide member (70,70'), and said needle guide member (70,70') is attached to a lower opening portion (50OD).

[13] The safety apparatus (1,1') for medical needles as recited in the above [12], wherein said needle guide member (70,70') has a nearly plate-shaped wall (71,71'),
   said wall (71,71') has a needle passage hole (72,72'), and
   said wall (71,71') has a needle guide member first latching portion (73,73') and a needle guide member second latching portion (75,75').

[14] The safety apparatus (1,1') for medical needles as recited in the above [12] or [13], wherein said fixing member (5,5') has a first side wall latching groove (52M) in the first side wall (52),
   said fixing member (5,5') has a needle guide member latching portion (60K) on the downward portion D side of the slide wall (60),
   the needle guide member first latching portion (73,73') of said needle guide member (70,70') is latched and kept fixed in said first side wall lathing groove (52M), and
   the needle guide member second latching portion (75,75') of said needle guide member (70,70') is latched and kept fixed in said needle guide member latching portion (60K).

[15] The safety apparatus (1,1') for medical needles as recited in any one of the above [1] to [14], wherein said needle (21) is bent toward the vertical axial line VL side at a predetermined angle with regard to the horizontal axial line HL somewhere in its length from the proximal end PE side to the distal end DE side, and
   the needle hub (22) on the proximal end PE side of said needle (21) is fixed to said shield hub (35).

Hereinafter, the first invention (embodiment) and the second invention (embodiment) will be generally referred to as "this invention", and when distinguished, these will be explained as "the first invention (embodiment)" and "the second invention (embodiment)".

### [Effect of the Invention]

This invention has the following advantageous effects.
According to the safety apparatus for medical needles according to the first invention (embodiment), when the first and second leg portions 31,32 extend from the first folded position to the second extended position, the wall 43DW of the slide member 42 is pulled into the space 40S of the connection hub 40H, whereby the wall 43DW of the slide member 42 blocks the cross section of space 40S of the connection hub 40H.
This constitution prevents the distal end 23 of the needle 21 from moving to a downward portion D lower than the wall 43DW of the slide member 42 and can reliably prevent the distal end 23 of the needle 21 from being exposed to an outside.

According to the safety apparatus for medical needles according to the second invention (embodiment), when the first and second leg portions 31,32 extend from the first folded position to the second extended position, the wall 42B' of the slide member 42' is pulled into the space 40S of the connection hub 40H', whereby the wall 42B' of the slide member 42' blocks the cross section of opening portion 40O' of the connection hub 40H'.
This constitution prevents the distal end 23 of the needle 21 from moving to a downward portion D lower than the wall 42B' of the slide member 42' and can reliably prevent the distal end 23 of the needle 21 from being exposed to an outside.

The first invention (embodiment) and the second invention (embodiment) produce the following advantageous effects in common.
That is, (i) even if the distal end 23 of the needle 21 has the blood, etc., of a patient adhering thereto, hygienically, the above-described constitution can prevent the blood, etc., from flying apart or sputter out of the connection hub 40H (or 40H'). Further, (ii) since that distal end 23 of the needle 21 which is contained in the connection hub 40H (or 40H') is never accidentally comes out of it, a worker can dispose of it free from anxiety. Furthermore, (iii) since the shield hub 35, leg portions 31,32 and connection hub 40H (or 40H') of the shield member 3 are formed from the same material by integral molding, they can be produced easily and can be produced inexpensively as compared with the apparatus described in Patent Document 1.

### [Brief Description of Drawings]

Fig. 1 is a general view (exploded perspective view) of a safety apparatus for medical needles according to this invention (first invention: the following Figures 1-23, 50-51 and 54-55 relate to the same first invention).
Fig. 2 is a general perspective view of a shield member viewed from the direction of a first side portion S1 and the direction of a proximal end PE.
Fig. 3 is a general perspective view of a shield member viewed from the direction of a first side portion S1 direction and the direction of a proximal end PE.
Fig. 4 is a partially enlarged view of a connection hub 40H when a shield member is viewed from the direction of a downward portion D and the direction of a distal end (DE).
Fig. 5 is a partially enlarged view of the connection hub 40H in Fig. 2.
Fig. 6 is a partially enlarged view of a connection hub 40H viewed from the direction of a downward portion D and from the direction of a first side portion S1.
Fig. 7 is general perspective view of a fixing member 5 before its assembling from the direction of an upper portion U and the direction of a distal end DE (a cover 51 is in the state of being open, and is also in the same state in the following Fig. 8).
Fig. 8 is a side view of the same in Fig. 7 from the direction of a first side portion S1.
Fig. 9 is a general perspective view of a fixing member 5 (which is in a first state or a second state) after its assembling from the direction of an upper portion U and the direction of a distal end DE (a cover 51 is in the state of being closed, and is also in the same state in the following Figs. 10-11 and 52-53).
Fig. 10 is a general perspective view of a fixing member 5 viewed from the direction of an upper portion U and the direction of a distal end DE.
Fig. 11 is a cross-sectional view taken along B-B' in Fig. 9.
Fig. 12 is a general perspective view of a safety apparatus for medical needles according to this invention (first invention) (which is in a first state: in a state where first and second leg portions (31,32) are in a completely folded position (first folded position) or a state before a needle is shielded).
Fig. 13 is a general perspective view of a safety apparatus for medical needles according to this invention (which is in a second state: in a state where first and second leg portions (31,32) are in a completely extended position (second extended position) or a state after a distal end 23 of a needle 21 is shielded).
Fig. 14 is a partially enlarged view of the safety apparatus in Fig. 12.
Fig. 15 is a partially enlarged longitudinal cross-sectional view of the safety apparatus in Fig. 12 in the direction of a horizontal axial line HL.
Fig. 16 is a partially enlarged lateral cross-sectional view of the safety apparatus in Fig. 12 in the direction of a first side portion S1 - a second side portion S2.
Fig. 17 is a partially enlarged longitudinal cross-sectional perspective view of the safety apparatus in Fig. 12 in the direction of a horizontal axial line HL.
Fig. 18 is a general perspective view of the safety apparatus in Fig. 12 viewed from the direction of a downward portion D and a second side portion S2.
Fig. 19 is a longitudinal cross-sectional view of the safety apparatus in Fig. 13 in the direction of a horizontal axial line HL.
Fig. 20 is a partially enlarged view of the safety apparatus in Fig. 19.
Fig. 21 is a partially enlarged cross-sectional view of a connection hub 40H and its vicinities in Fig. 13 in the direction of a first side portion S1 - a second side portion S2.
Fig. 22 is a partially enlarged longitudinal (cross-) sectional view of a connection hub 40H and its vicinities in Fig. 13 in the direction of a horizontal axial line HL viewed from the direction of a second side portion S2.
Fig. 23 is a general perspective view of the safety apparatus in Fig. 13 viewed from the direction of d downward portion and a proximal end PE.
Fig. 24 is a general perspective view of a safety apparatus for medical needles according to this invention (which is a second invention, and Figs 24-49 and 52-53 relate to the same second invention) (first state: it is in a state where first and second leg portions (31,32) are in a completely folded position (first folded position) or in a state before the needle is shielded).
Fig. 25(A) is a general perspective view of the safety apparatus in Fig 1 viewed from the direction of a downward portion D and a proximal end (PE) side. Fig. 25(B) is a partially enlarged view of the same in Fig. 25(A).
Fig. 26(A) is a plan view of the safety apparatus in Fig. 1. Fig. 26(B) is a bottom view of the same in Fig. 1.
Fig. 27(A) is a front view of the safety apparatus in Fig. 1 viewed from a distal end DE side. Fig. 27(B) is a front view of the same in Fig. 24 viewed from a proximal end PE side.
Fig. 28(A) is a side view of the safety apparatus in Fig. 24 viewed from a first side portion S1 side. Fig. 28(B) is a side view of the same in Fig. 24 viewed from a second side portion S2 side.
Fig. 29(A) is a longitudinal cross-sectional view of the safety apparatus in Fig. 24 in a horizontal axial line. Fig. 29(B) is a partially enlarged view of the same in Fig. 29(A).
Fig. 30 is a general perspective view of a safety apparatus for medical needles according to this invention (second state: a state where the first and second leg portions (31,32) are in a completely extended state (second extended position) or a state after the distal end 23 of a needle 21 is shielded).
Fig. 31(A) is a general perspective view of the safety apparatus in Fig. 30 viewed from the direction of a downward portion D and from a proximal end PE side. Fig. 31(B) is a partially enlarged view of the safety apparatus in Fig. 31(A).
Fig. 32 is a general perspective view of the safety apparatus in Fig. 30 viewed from the direction of an upper portion U and from a proximal end PE side.
Fig. 33(A) is a plan view of the safety apparatus in Fig. 30. Fig. 33(B) is a bottom view of the safety apparatus in Fig. 30.
Fig. 34 is a rear view of the safety apparatus in Fig. 30. Fig. 34(B) is a side view of the safety apparatus in Fig. 30 viewed from a first side portion S1 side. Fig. 34(C) is a side view of the same viewed from a second side portion S2 side.
Fig. 35 is a longitudinal cross-sectional view of the safety apparatus in Fig. 30. Fig. 35(B) is a partially enlarged view of the safety apparatus in Fig. 35(A).
Fig. 36 is a general view of a safety apparatus for medical needles according to this invention (second invention) (an exploded perspective view of the same before it is assembled).
Fig. 37(A) is a general perspective view of a shield member viewed from the direction of an upper portion U and a distal end DE side before its assembling. Fig. 37(B) is a front view of the shield member. Fig. 37(C) is a general perspective view of the shield member viewed from the direction of a downward portion D and a distal end DE side.
Fig. 38(A) is a partially enlarged view of a connection hub 40H and its vicinities in Fig. 37(A). Fig. 38(B) is a partially enlarged view of the connection hub 40H and its vicinities viewed from the direction of a downward portion D and a proximal end PE side.
Fig. 39(A) is a general perspective view of a shield member viewed from the direction of an upper portion U and a proximal end side before its assembling. Fig. 39(B) is a rear view of the shield member. Fig. 39(C) is a general perspective view of the shield member viewed from the direction of a downward portion D and a proximal end PE side.
Fig. 40(A) is a plan view of a shield member before its assembling. Fig. 40(B) is a side view of the shield member in Fig. 40(A) viewed from a first side portion S1 side. Fig. 40(C) is a bottom view of the shield member in Fig. 40(A).
Fig. 41 is a general perspective view of a fixing member 5 before its assembling (a cover 51 is in a state of being open and is in the same state in Figs. 42 to 45) viewed from the direction of an upper portion U and from a distal end DE side.
Fig. 42 is a general perspective view of the fixing member 5 viewed from the direction of an upper portion U and from a proximal end PE side.
Fig. 43(A) is a general perspective view of the fixing member 5 viewed from the direction of a downward portion D and from a distal end DE side. Fig. 43B is a general perspective view of the fixing member 5 viewed from the direction of a downward portion D and from a proximal end PE side.
Fig. 44(A) is a plan view of the fixing member 5. Fig. 44(B) is a bottom view of the fixing member 5.
Fig. 45(A) is a front view of the fixing member 5. Fig. 45(B) is a side view of the fixing member 5 viewed from a first side portion S1 side. Fig. 45(C) is a cross-sectional view taken along B-B in Fig. 45(B). Fig. 45(D) is a rear view of the fixing member 5.
Fig. 46(A) is a general perspective view of the fixing member 5 (in a first state or second state) after its assembling (a cover 51 is in a state of being closed and also in the same state in Figs. 47 to 49) viewed from the direction of an upper portion U and from a distal end DE side. Fig. 46(B) is a general perspective view of the fixing member 5 viewed from the direction of an upper portion U and from a proximal end DE side.
Fig. 47(A) is a general perspective view of the fixing member 5 viewed from the direction of a downward portion and from a distal end DE side. Fig. 47(B) is a general perspective view of the fixing member 5 viewed from a downward portion D and from a proximal end PE side.
Fig. 48(A) is a plan view of the fixing member 5. Fig. 48(B) is a bottom view of the fixing member 5.
Fig. 49(A) is a front view of the fixing member 5. Fig. 49(B) is a side view of the fixing member 5 viewed from a first side portion S1 side. Fig. 49(C) is a cross-sectional view taken along B-B in Fig. 49(B). Fig. 49(D) is a rear view of the fixing member 5.
Fig. 50 is a general perspective view of a medical safety apparatus having a needle guide member 70 fit in a lower opening portion 50OD of a fixing member 5' viewed from a distal end DE side and from a downward portion D.
Fig. 51 is a general perspective view of the needle guide member 70 viewed from a distal end DE side and from the direction of an upper portion U.
Fig. 52 is a general perspective view of a needle guide member 70' (other embodiment) viewed from a distal end side and from the direction of an upper portion U.
Fig. 53 corresponds to Fig. 29, and Fig. 53(A) is a longitudinal cross-sectional view of a fixing member 5' (of the second invention (embodiment)) in which a needle guide member latching portion 60K (latching groove) is formed in a slide wall 60. Fig. 53(B) is a longitudinal cross-sectional view of the fixing member 5' in which a needle guide member 70 is fit in a lower opening portion 50OD.
Fig. 54 corresponds to Fig. 19, and Fig. 54(A) is a longitudinal cross-sectional view of a fixing member 5 (of the first invention (embodiment)) in which a needle guide latching portion 60K (latching groove) is formed in a slide wall 60. Fig. 54(B) is a longitudinal cross-sectional view of a fixing member 5 in which a needle guide member 70 is fit in a lower opening portion 50OD.
Fig. 55 corresponds to Fig. 7 and is a general perspective view of a fixing member 5 (of the first invention (embodiment)) in which a first sidewall latching groove 52M is formed on a first sidewall 52.

### [Preferred Embodiments of the Invention]

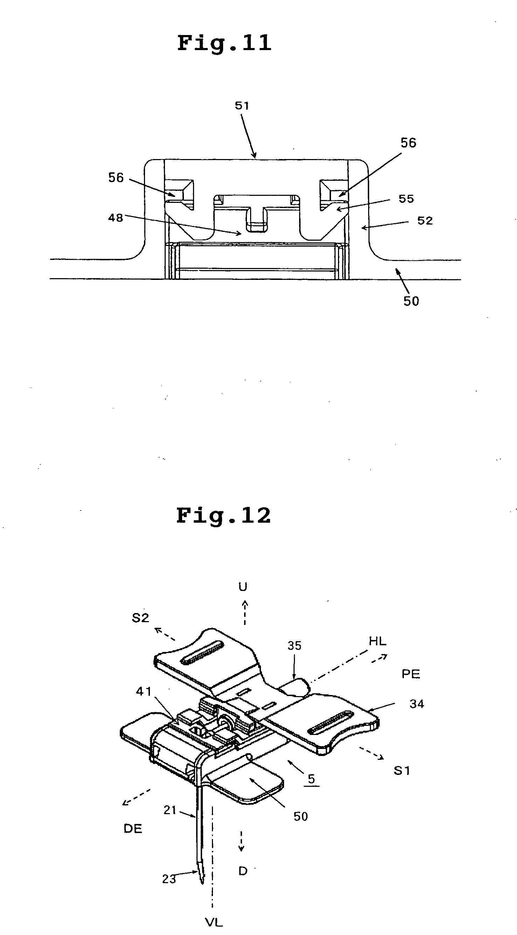

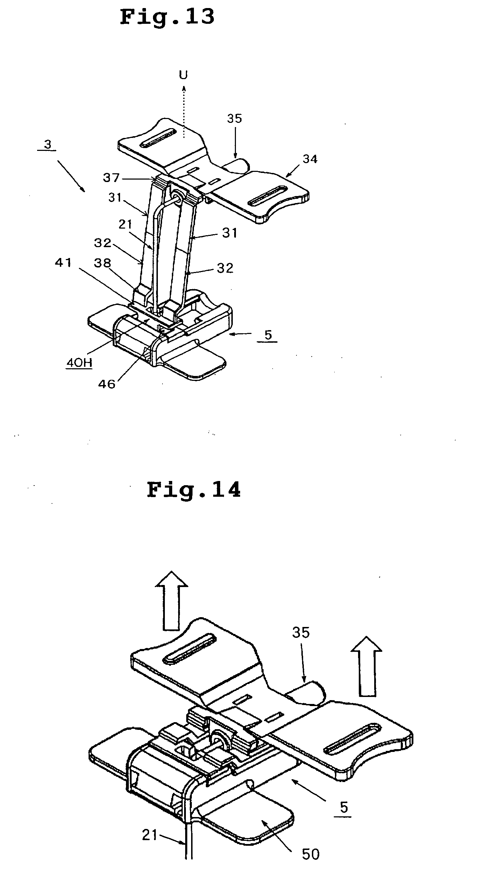

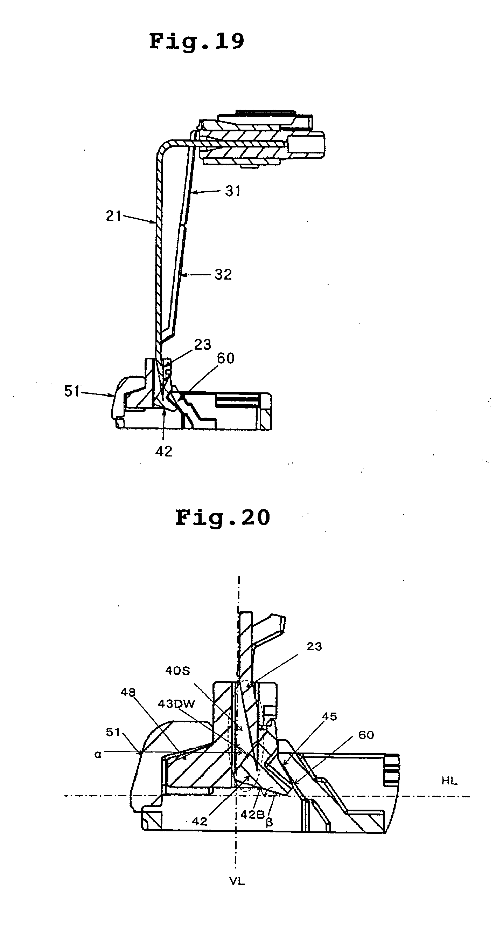

This invention will be explained in detail with reference to drawings hereinafter.
The following definitions are made for giving a clear explanation of this invention.
(i) In the safety apparatus for medical needles in this invention, "a first state" (first folded position) refers to a state wherein the extendable first and second leg portions (31,32) of a shield member 3 shown in Figs. 2-3 are in a completely folded position, for example, as shown in Fig. 12. In other words, it means that the first and second leg portions (31,32) are in a completely folded state, that a needle 21 extends downwardly and is in a state where it is operated for transfusion or in use, and further that a distal end 23 is not protected (unshielded).
(ii) Similarly, "a second state" (second folding position) refers to a state where the first and second leg portions (31,32) of a shield member 3 are in a completely extended position, for example, as shown in Figs. 13 and 19. In other words, the usage of a needle (puncture) is finished, and the distal end 23 of a needle 21 is confined or retreated in the present safety apparatus for needles and protected (shielded) in a state where the first and second leg portions (31, 32) are in a completely extended state.
(iii) "a transition state" means a state in transition from "a first state" to "a second state".
(iv) "a transition start state" means an initial state at which transition starts from "a first state" to "a second state".
(v) "a transition end state" means a final state at which transition from "a first state" to "a second state" ends.
(vi) "a proximal end PE (side or direction)" in this invention means an end portion of a tube T side to be connected to a needle hub 22 as shown in Figs. 1 and 12.
(vii) "a distal end DE(side or direction)" means an end portion of a side where a needle 21 is fit as shown in Figs. 1 and 12.
(viii) "a horizontal axial line HL (direction)" means a direction in which, for example, a tubular needle hub 22 extends longitudinally as shown in Figs. 1 and 12.
(iix) "a vertical axial line VL (direction)" means a direction in which, for example, a bent distal end 23 side of a needle 21 extends vertically downward as shown in Figs. 1 and 2 (see a dashed line in Figs. 1 and 12).
(ix) "a first side portion S1 (side or direction)" means, for example, a direction of right side end portion of a wing 34 as shown in Figs. 1 and 12 (see an arrow with a dashed line in Figs. 1 and 12).
(x) "a second side portion S2 (side or direction)" means, for example, a direction of left side end portion of the wing 34 as shown in Figs. 1 and 12 (see a dashed line in Figs. 1 and 12).
(xi) "upper portion U (side or direction)" means, for example, a direction opposite to a bent distal end 23 side of a needle 21 as shown in Figs. 1 and 12 (see an arrow with a dashed line in Figs. 1 and 12).
(xii) "a downward D (side or direction)" means, for example, a direction of a distal end 23 side of a needle 21 as shown in Figs. 1 and 12 (see an arrow with a dashed line in Figs. 1 and 12).

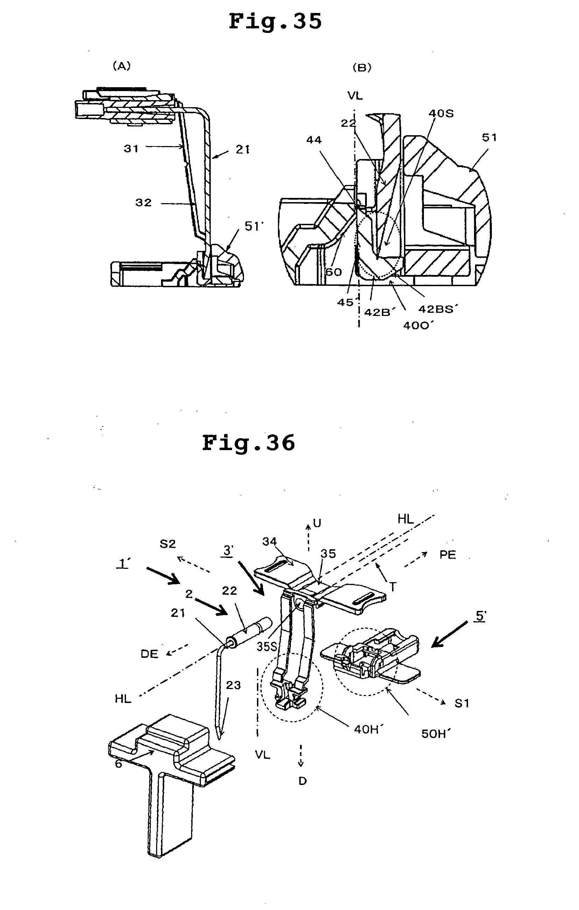

In the present invention, as is shown by general figures of Fig.1 and Fig.36 representing its embodiments, shield member 3,3' and fixing member 5,5' are generally same in shape and only differ in shape with respect to connection hug 40H,40H', or part of the constitution of the fixing member 5,5'.
First, the first embodiment of the invention will be explained below.

### [Safety apparatus 1 for medical needles]

The safety apparatus 1 for medical needles (to be sometimes simply referred to as "apparatus 1" hereinafter) according to this invention will be explained with reference to drawings.
The safety apparatus 1 for medical needles according to this invention has a needle member 2, a shield member 3 and a fixing member 5 as shown in Figures.
The form and function of the needle member 2, shield member 3 and fixing member 5 are as described below.

### [Needle member 2]

The needle member 2 has a needle 21 and a needle hub 22.
As shown in Fig. 1, the needle 21 is fixed to one end of the needle hub 22 and is bent toward a vertical axial line VL side at a predetermined angle (e.g., approximately 90 degrees) with regard to a horizontal axial line HL somewhere along the line from a proximal end PE side to a distal end DE side.
The needle 21 has a distal end 23 in a downward portion D. The distal end 23 has an acuate pointed end on the downward portion D side.
The distal end 23 is bent at a slight predetermined angle toward the proximal end PE side at a slight angle with regard to the vertical axial line VL so that the puncture procedure is practiced smoothly and stably.
The proximal end PE side of the needle 21 is fit (fixed) in a tubular needle hub 22.
The distal end 23 of the needle 21 is normally used to make a puncture in an infusion port (not shown) implanted in a patient's body. A medicinal reagent solution is injected into a patient through the distal end 23 from a tube T (shown by a dash line in Fig. 1) connected to the proximal end PE side of the needle hub 22. The tube T is normally connected to a medicinal reagent solution bag, and the reagent solution is supplied from the bag.
After the injection of the medicinal reagent solution into a patient is completed, the needle 21 is pulled out of the infusion port (not shown).

### [General description of shield member 3]

The shield member 3 is a member that receives a needle 21 which has been used to inject a medicinal reagent solution into a patient's body and protects (shields) the distal end 23 of the needle from exposure. The shield member 3 has a shield hub 35, leg portions (31,32) and a shield member connection hub 40H (to be sometimes abbreviated as "connection hub 40H" hereinafter). The shield hub 35 receives and fixes the needle hub 22.
The leg portions (31,32) are composed of at least 2 leg portions (31,32). The leg portions (31,32) are bendably connected to each other with an intermediate hinge 39. That is, they have a constitution in which a first leg portion 31 constituting an upper half and a second leg portion 32 constituting a lower half are folded and extended through the above intermediate hinge 39 such that they extend from a first folded position to a second extended position.
After the injection of a medicinal reagent solution into a patent's body is completed, the connection hub 40H in the shield member 3 receives the distal end 23 of the needle 21 therein, confines it therein and protects (shields) it.

### [General description of fixing member 5]

The fixing member 5 receives the shield member 3 and fixes it when the needle is used, for example, as shown in Fig. 12, and more specifically, it holds both the connection hub 40H and the shield hub 35, and after the needle is once used, it holds only the connection hub 40H, for example, as shown in Fig. 13. (The shield hub 35 is lifted up to an upper portion, being released from the fixing member 5 and thereby extending the leg portions, whereby the top portion of the needle that is lifted up is protected (shielded) within the connection hub 40H.)
That is, the fixing member 5 is first connected to the connection hub 40H of the shield member 3 to fix the connection hub 40H.
When the first and second leg portions (31, 32) are in a first folded position (first state), the fixing member 5 contains the first and second leg portions (31,32), the shield member 3 and the needle hub 22.
Further, the fixing member 5 is a member that is fixed (fit) to a patient's skin when used (a needle is used to make a puncture), by harnessing a flat contact surface plate 50.

### [Explanations of parts of shield member 3]

The shield member 3 has the shield hub 35, the shield member connection hub 40H and the first and second leg portions (31,32) as shown, for example, in Fig.s 2-3.
The shield hub 35 is formed relatively on the proximal end PE side of the shield member 3, and the shield member connection hub 40H is formed relatively on the distal end DE side thereof. The first and second leg portions (31,32) are formed between the proximal end PE side and the distal end DE side.
The shield hub 35 and the shield member connection hub 40H are connected to each other through the first and second leg portions (31,32) as shown in Fig. 1, etc.

### [Shield hub 35]

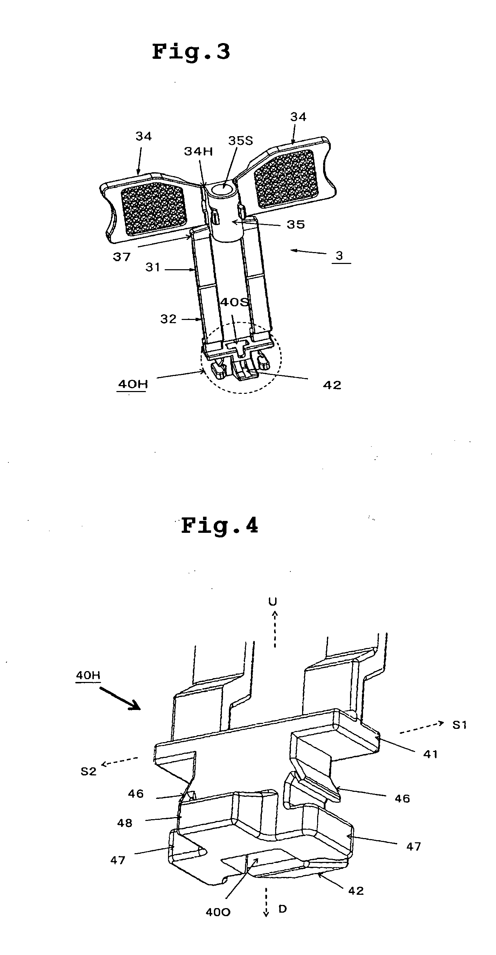

The shield hub 35 is a hub that is to fix the needle hub 22. The Shield hub 35 works as a member that receives or accommodates the needle hub 22.
The shield hub 35 is formed in a nearly tubular form (to be sometimes described as "nearly cylindrical form").
The shield hub 35 may have any form so long as it can receive the needle hub 22 and fix it in this state. For example, it has a nearly square-shaped tubular form.
The shield hub 35 has a distal end DE side to which the first and second leg portions (31,32) through a proximal end side hinge 37.
The shield hub 35 has a first side portion S1 to which wing 34 is connected and a second side portion S2 to which wing 34 is connected, respectively. The wings 34 and 34 work as handles to lift up the needle 21 (received in the shield hub 35 and fixed therein) toward an upper portion U.
The wings 34 and 34 and the shield hub 35 are connected through hinges 34H as shown in Figs. 2 and 3, whereby the first side portion S1 and the second side portion S2 of the wings 34 and 34 are manually picked up, the wings 34 and 34 are folded toward an upper portion U and the first and second leg portions (31,32) are extended from the first folded position to the second extended position, when the needle 21 can be lifted up toward an upper portion U as shown in Fig. 13.
It is added further that (nearly rectangular) concavo-convex portions (not indicated by any symbols) are formed on the upper portions U of the wings 34 and 34 of the first side portion S1 and the second side portion S2. The wings 34 and 34 are folded together to cause these concavo-convex portions to engage with each other, and the needle 21 can be lifted up toward an upper portion U.

### [First and second leg portions (31,32)]

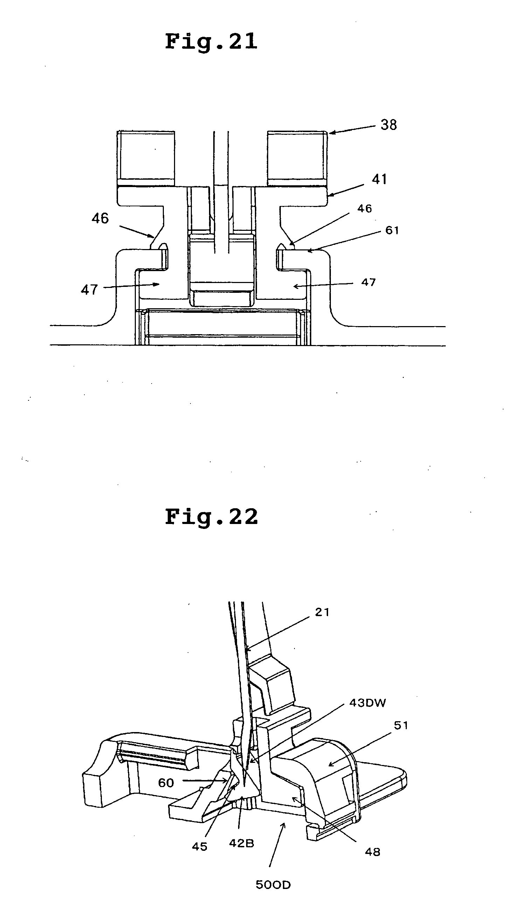

The first and second leg portions (31,32) are formed such that they can extend from the first folded position (first state) to the second extended position (second state).
When the first and second leg portions (31,32) extend from the first folded position to the second extended position, the slide member 42 of connection hub 40H of the shield member and the needle 21 cooperatively move as shown in Figs. 12-13. The first and second leg portions (31,32) have a function of so-called "tow rope" to the slide member 42 and the needle 21.
When the first and second leg portions (31,32) finishes extending up to the second extended position, the distal end 23 of the needle 21 and the distal end side wall 43DW of the slide member 42 (see Fig. 22) are pulled into (and accommodated in) the space 40S of the shield member connection hub 40H as shown in Fig. 20.
The first and second leg portions (31,32) are formed in such a shape that the two members are capable of extending from a folded form as is illustrated in Figs. 1 and 13.
The first leg portion 31 is arranged relatively on the proximal end PE side of the shield member 3. The second leg portion 32 is arranged relatively on the distal end DE side thereof.
The first and second leg portions (31,32) are formed on the first side portion S1 side, and the same first and second leg portions (31,32) are also formed on the second side portion S2 side to form a pair. The first and second leg portions (31,32) are connected to the distal end DE side of the shield hub 35 through the proximal end side hinges 37.
The first leg portions 31, 31 are connected to the distal DE side of shield hub 35 through proximal end side hinges 37.
The second leg portions 32 and 32 are connected to the upper portion U side of upper connection portion 41 of the connection hub 40H through distal end side hinges 38.
The first leg portion 31 and the second leg portion 32 are connected to each other through an intermediate hinge 39 as described above.

In the above explanation of this invention, the "leg portion" is described as "first and second leg portions (31,32), and they have a form capable of extending from their folded form, while it may be composed of two or more members which are capable of extending from their folded state.
The requisite is that the leg portion is to be constituted in a form (with a shape, a structure and a material) capable of bringing the "first state" (a state where the leg portion is in a completely folded state, in other words, a state where the distal end 23 of the needle 21 is kept unprotected (unshielded) in a state where the leg portion is completely folded, and for example, see Fig. 12, etc.) into the "second state" (a state where the leg portion is in a completely extended state, in other words, a state wherein the distal end 23 of the needle 21 is kept protected (shielded) in a state where the leg is completely extended, and for example, see Figs, 13, 19, etc).

### [Connection hub 40H of shield member (Explanations of members)]

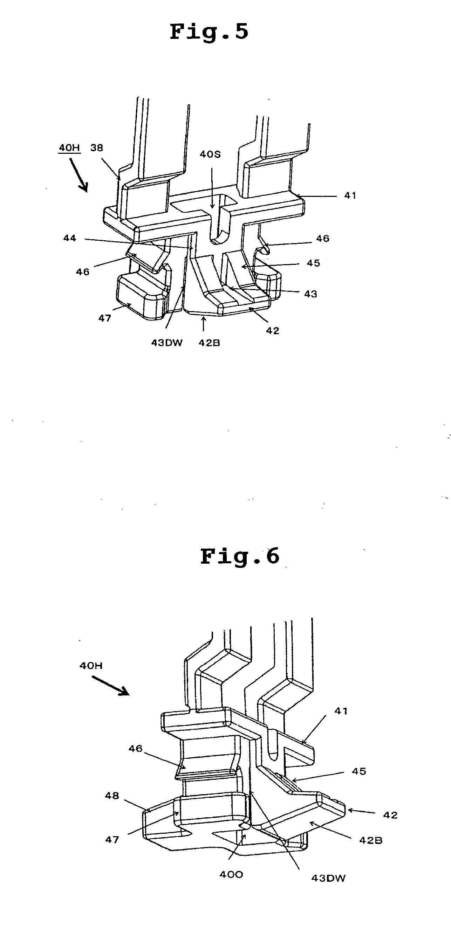

The connection hub 40H is a member that is connected to the fixing member connection hub 50H (to be sometimes abbreviated as "connection hub 50H" hereinafter) of the fixing member 5.
The above connection hub 40H is a member that takes the distal end 23 of the needle 21 and the distal end side wall 43DW of the slide member 43 to be described later as shown in Fig.5 into the space 40S and protects (shields) the needle's distal end, where the leg portion [first and second leg portions (31,32)] is in completely extended state as shown in Fig.13.
As illustrated in Figs. 4, 5 and 6, the connection hub 40H of the shield member has the slide member 42, the shield member first latching portion (48) (to be sometimes abbreviated as "first latching portion 48" hereinafter), the shield member second latching portion 46 (to be sometimes abbreviated as "second latching portion 46" hereinafter) and the shield member third latching portion 47 (to be sometimes abbreviated as "third latching portion 47" hereinafter).
The connection hub 40H of the shield member has the slide member 42 formed on the proximal end PE side and its first latching portion 48 formed on the distal end DE side.
As shown in Fig. 4, further, the connection hub 40H has the first side portion S1 side and the second side portion S2 side where the above second latching portion 46 and third latching portion 47 are formed (protruded).
The above second latching portion 46 is formed between the above portion connection portion 41 and the third latching portion 47. In other words, the second latching portion 46 is formed in a downward portion D of the above portion connection portion 41 and in an upper portion U of the third latching portion 47. The slide member 42 and the first latching portion 48 and third latching portion 47 thereof are formed in substantially nearly the same level positions when viewed in the direction of a horizontal axial line HL.

The connection hub 40H of the shield member has a space 40S formed nearly in the middle as illustrated in Figs. 2, 3 and 5 and has an opening portion 400 in a downward portion D as illustrated in Figs. 4 and 6.
The space 40S and the opening portion 400 work as a passage for the needle 21 and the distal end 23. The space 40S made through from an upper portion U of the above connection hub 40H to the downward portion D thereof.
The space 40S is formed so as to have a size (volume) capable of containing or accommodating at least the distal end 23 of the needle 21 (or part of the distal end 23) and the distal end side wall 43DW of the slide member 42 (see Fig. 22) (or part of the distal end side wall 43DW, at least that portion which blocks the cross section of the space 40S).

Further, the above connection hub 40H has an upper portion connection portion 41 formed in an upper portion U. The upper portion connection portion 41 has nearly the form of a plate (also called "the form of a sword guard or flange") as illustrated in Fig. 4.
The upper portion connection portion 41 is connected to the first and second leg portions (31,32) through a distal end side hinge 38 (see Figs. 1, 2, 5, etc.).
When the first and second leg portions (31,32) extend from the first folded position to the second extended position, the slide member 42 of connection hub 40H of the shield member thereby performs a so-called "swing" to shift in position from the proximal end PE side to the distal end DE side cooperatively with the first and second leg portions (31,32) through the hinge 44 as will be described later.

### [(First) slide member 42]

The slide member 42 is basically a member having the distal end side wall 43DW, the slide wall 45 (to be also referred to as inclined wall 45), a downward portion wall 42B and a groove 43. The slide member 42 of the first invention is sometimes designated as first slide member 42.
The above slide member 42 is connected to the connection hub 40H through the hinge 44 as shown in Fig. 5, so that it can shift in place (swing) from the proximal end PE side to the distal end DE side with the above hinge as a start point.
The above action (movement) will be explained more specifically. When the first and second leg portions (31,32) extend from the first folded position to the second extended position, the slide member 42 connected to the connection hub 40H with a hinge moves cooperatively therewith.
That is, when the first and second leg portions (31,32) extend from the first folded position to the second extended position, the slide member 42 "swings" from a position in Fig. 15 (first position) to a position in Fig. 20 (second position) with the hinge 44 as a start point (or a center) to shift in position so as to block the cross section of space 40S of the connection hub 40H.

Further, the "first position" of the slide member 42 means that the distal end side wall 43DW is in a position in Fig. 15 and in a position where it keeps blocking no cross section of space 40S of the connection hub 40H.
Furthermore, it means a position where the downward portion wall 42B keeps blocking no opening portion 400 (see Figs. 4, 6, etc.) of the connection hub 40H (or vicinities of the opening portion 400). On the other hand, the "second position" of the slide member 42 means a position where the distal end side wall 43DW is in a position shown in Fig. 20 and blocks the cross section of space 40S of the connection hub 40H. Further, it means a position where the downward portion wall 42B blocks the cross section of opening portion 400 (or vicinities to the opening portion 400) of the connection hub 40H.
The slide member 42 has the distal end side wall 43DW, the slide wall 45 (to be also referred to as "inclined wall 34"), the downward portion wall 42B and the groove 43. More specifically, the slide member 42 nearly has the form of the letter L formed of the distal end side wall 43DW and the downward portion wall 42B when viewed from the direction of the first side portion S1 (or the second side portion S2).

Further, a pair of "inclined walls 45" is formed as "slide wall 45" in the direction of the first side portion S1 and the second side portion S2 having nearly the form of the letter L as shown in Figs. 5-6.
In the first state ("first position" of the slide member 42") after assembling, the slide wall (inclined wall) 45" is nearly in parallel with the vertical axial line VL as illustrated in Fig. 15. The downward portion walk 428 is nearly in parallel with the horizontal axial line HL.
In the second state ("second position" of the slide member 42") after assembling, the slide wall 45 is arranged to come across the vertical axial line VL at an acute angle of α as illustrated in Fig. 20. In other words, it is arranged to be slanted as it goes down from the proximal end PE side to the distal end DE side. The downward portion wall 42B is arranged to come across the horizontal axial line HL at an acute angle of β. In other words, it is arranged to be slanted as it goes up from the proximal end PE side to the distal end DE side.
As shown in Fig. 20, when the first and second leg portions (31,32) extend from the first folded position to the second extended position, the distal end side wall 43DW is pulled into the space 40S of the connection hub 40H cooperatively therewith to block the cross section of the space 40S. The downward portion wall 42B also cooperatively therewith blocks the cross section of opening portion 400 of the connection hub 40H) (or vicinities of the opening portion 400).

### [Slide wall 45 (inclined wall 45)]

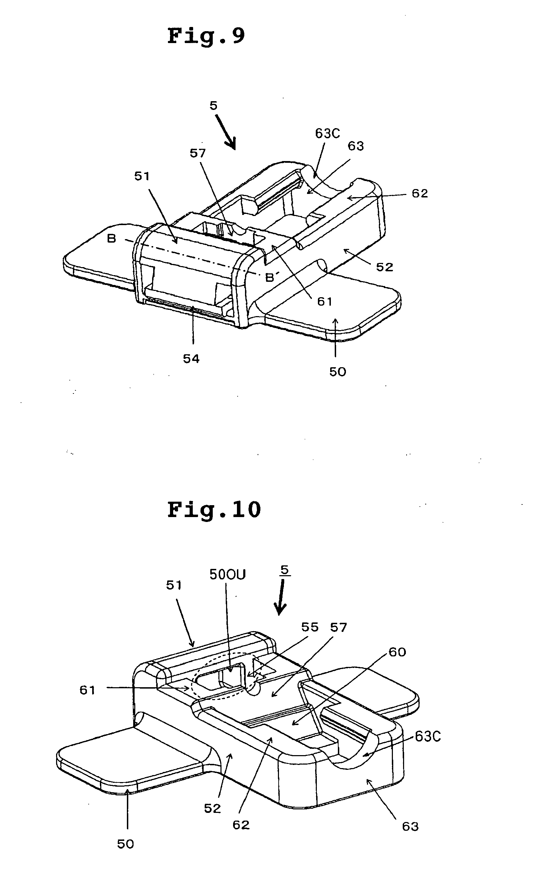

The slide wall 45 is a member that comes in contact with the slide wall 60 (to be also described as "inclined wall 60") (see Fig. 10) of connection hub 50H of the fixing member 5.
A case where the "inclined wall 45" is used as the slide wall 45 will be explained below.
Inclined walls 45 are formed as opposed to each other on the first side S1 side and the second side S2 side (a pair of them is formed).
The inclined walls 45 have inclined surfaces that go up from the proximal end PE side to the distal end DE side. The inclined surfaces have step portions somewhere in the middle.
When the first and second leg portions (31,32) extend from the first folded position to the second extended position, the inclined surfaces of the inclined walls 45 cooperatively therewith move (or perform so-called "sliding") from the proximal end PE side to the distal end DE side, wherein the inclined surfaces are kept in contact with the inclined surface of inclined wall 60 of connection hub 50H of the fixing member 5. The distal end side wall 43DW is thereby facilitated to be pulled into the space 40S of connection hub 40H of the shield member. The downward portion wall 42B also facilitatively blocks the cross section of the opening portion 400 (or vicinities of the opening portion 400) of the connection hub 40H.
The slide member 42 is a member that is latched and fixed by the third latching portion 58 of the fixing member 5. The slide member 42 has the groove 43 formed between the two inclined walls 45. The groove 43 works or functions as a latching groove to latch the third latching portion 58 of connection hub 50H of the fixing member 5 to be described later.
The groove 43, being latched with the third latching portion 58, prevents the connection hub 40H from being pulled out of the connection hub 50H of the fixing member 5.

When the slide member 42 is in the first state [when the first and second leg portions (31,32) are in the first folded position as shown in Fig. 15], the distal end side wall 43DW of the slide member 42 is free from blocking the cross section of the space 40S of the connection hub 40H. The downward portion wall 42B is free from blocking the cross section of opening portion 400 (or vicinities of the opening portion 400) of the connection hub 40H. (The needle 21 is hence protruded to a downward portion through the opening portion 400).
In the first state, the needle 21 is inserted into the space 40S of the connection hub 40H and extends far downward through it (see Figs. 15, 17 and 18).
In the second state [when the first and second leg portions (31,32) are in the second extended position], the distal end side wall 43DW of the slide member 42 is pulled into the space 40S of the connection hub 40H as shown in Fig. 20.
The distal end side wall 43DW of the slide member 42 thereby blocks the cross section of space 40S of the connection hub 40H, and further, the downward portion wall 42B blocks the cross section of opening portion 400 (or vicinities of the opening portion 400) of the connection hub 40H, and prevents the distal end 23 of the needle 21 from moving to a downward portion D lower than the distal end side wall 43DW of the slide member 42.
This constitution can prevent the distal end 23 of the needle 21 from being exposed from the opening portion 400 of the connection hub 40H (see Fig. 20).

### [First latching portion 48, second latching portion 46 and third latching portion 47 of shield member]

Latching of the connection hub 40H of the shield member 3 and the fixing member 5 will be explained below. The fixing member 5 is connected to the shield member 3 through or via the connection hub 40h as shown in Fig. 13. In this state, the first latching portion 48, second latching portion 46 and third latching portion 47 of the shield member 3 are members that work of function to prevent the above connection hub 40H from being pulled out of the connection hub 50H of the fixing member 5 when the first and second leg portions (31,32) extend from the first folded position to the second extended position.

The first latching portion 48 of the shield member 3 has the form of what is called "protruded" and a nearly trapezoidal form as illustrated in Fig. 4 (nearly the form of protruding in a trapezoidal shape).
When the first and second leg portions (31,32) extend from the first folded position to the second extended position, the above first latching portion 48 is cooperatively therewith pulled in the direction of an upper portion U to be latched and kept fixed by the downward portion D of wall surface 51W of cover 51 of the fixing member 5. This constitution can prevent the connection hub 40H of the shield member from being pulled out of the connection hub 50H of the fixing member 5 ("second state") (see Fig. 22).

The second latching portion 46 of the shield member 3 has the form of what is called "a hook (nail)" as is illustrated in Fig. 4. It is formed (protruded) in the directions of the first side portion S1 and the second side portion S2 from the center of the connection hub 40H as it is inclined in the direction of the downward portion D (a pair of the second latching portions 46,46 is formed).
When the first and second leg portions (31,32) extend from the first folded position to the second extended position, the second latching portion 46 cooperatively therewith moves toward the upper portion U and goes over the first upper wall 61 of connection hub 50H of the fixing member 5 to latch onto the upper portion (top surface) of the upper wall 61 as shown in Fig. 21. The second latching portion 46 works as a so-called "return-prevention hook" in this manner (see Fig. 21).
Further specifically, when the above second latching portion 46 of the shield member goes or gets over the first upper wall 61, while moving from the first state to the second state, a worker handling the apparatus can sense a latching feel when it goes over the first upper wall 61. Thus, the latching process has a function of a signaling the state wherein the distal end 23 of the needle 21 has been pulled into the connection hub 40H to be protected (shielded), telling that prevention of so-called wrong puncture is completed.

On the other hand, the third latching portion 47 of the shield member 3 has the form of a so-called "sword guard (flange)" as illustrated in Fig. 4. (A pair of the third latching portions 47, 47 is formed.)
When the first and second leg portions (31,32) finish extending from the first folded position to the second extended position, the above third latching portion 47 is latched and kept fixed by the lower portion (bottom surface) of the upper wall 61 as shown in Fig. 21. That is, the upper portion of the third latching portion 47 works as "a breakaway (pullout) preventing stopper".
In the shield member, the first latching portion 48 having the form of a trapezoid, the second latching portion 46 having the form of a hook" and the third latching portion 47 having the form of a flange keep the shield member from coming out of the connection hub 50H of the fixing member 5 in the above manner, and maintain the "second state" shown, for example, in Fig. 13.

### [Fixing member 5]

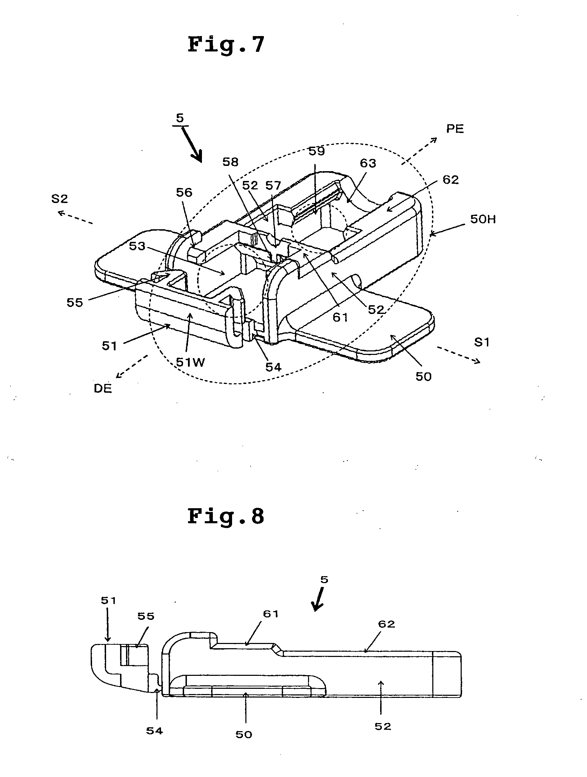

The fixing member 5 generally has the connection hub 50H that is a portion to be connected to the shield member 5 and a flat contact surface plate 50 to be fixed to (an outer skin of the body of) a patient. The connection hub 50H is a member that is connected to the connection hub 40H of the shield member 3.
The connection hub 50H has the following form and structure. As shown in Fig.7, for example, the connection hub 50H has a plurality of walls (first side wall 52, intermediate wall 57 and second side wall 63) and there connected the cover 51 on a distal end DE side.

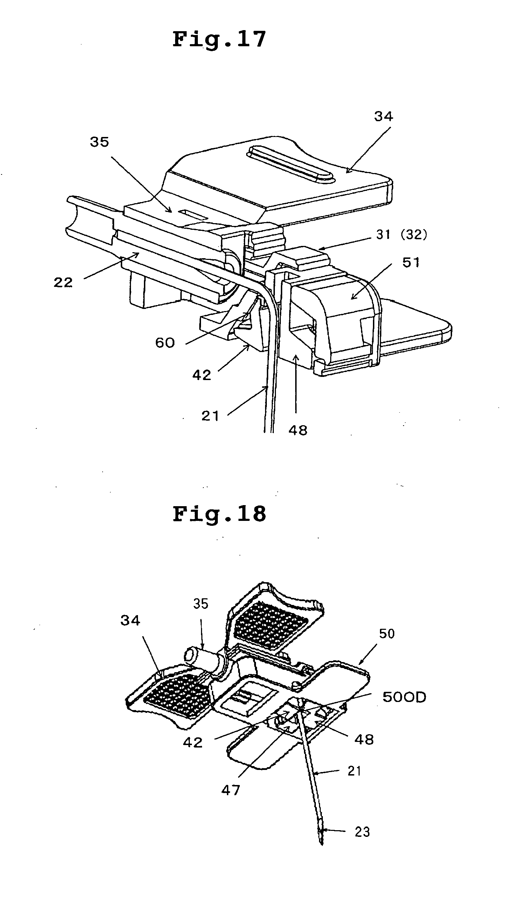

The connection hub 50H has, connected there to, contact surface plates 50,50 to be fixed to a patient on each of the first side portion S1 on the distal end DE side and the second side portion S2 side, formed for example by integral molding method.
Spaces (53, 59) for receiving the shield member 3 are formed with the wall 51W of the above cover 51 and the above plurality of walls (52, 57, 63) as shown in Fig. 7.
These spaces (53,59) are, more specifically, sectioned into the first space 53 and the second space 59.
The first space 53 in the fixing member 5 has an upper opening portion 50OU (see Fig. 10) and a lower opening portion 50OD (see Fig. 18). The upper opening portion 50OU and the lower opening portion 50OD make an open pass-through communicating each other as shown in Fig. 7. (The upper opening portion 50OU and the lower opening portion 50OD allow the needle 21 to extend downwardly as shown in Fig.18.)

### [Cover 51 and first latching portion]

The cover 51 of the fixing member 5 is a member for fixing the connection hub 40H of the shield member 3 to the connection hub 50H of the fixing member 5.
The cover 51 has a form (shape and structure) as illustrated in Fig. 7. The cover 51 has a wall 51W having nearly the form of a plate (rectangle) as shown in Fig. 7, and has a first latching portion 55 that is protruded from the distal end DE side to the proximal end PE side of the connection hub 50H of the fixing member 5.
The downward portion D of the cover 51 is connected to the distal end DE side of the connection hub 50H of the fixing member through a hinge 54.
The cover 51 is formed such that it turns at about 90° to the proximal end PE side through the hinge 54 so that the first latching portion 55 of the fixing member 5 (to be sometimes abbreviated as "first latching portion 55" hereinafter) and the second latching portion 56 of the fixing member 5 to be described later, and to be sometimes abbreviated as "second latching portion 56" hereinafter) can latch onto each other as illustrated in Figs. 7 to 11.
The first latching portion 55 of the fixing member 5 has the form of a so-called "wedge", and a pair of the latching portions 55 is protruded from the distal end DE side to the proximal end PE side of the cover 51 as illustrated in Figs. 7 to 11.
Further, the first latching portion 55 of the fixing member 5 is formed in the form of a so-called "wedge" as described above such that it can latch onto the second latching portion 56 formed inside the first side wall 52.

The first space 53 shown in Fig. 7 is provided for containing or accommodating the connection hub 40H of the shield member 3 and fixing it.
On the other hand, the second space 59 is provided for receiving the shield hub 35 of the shield member 3, the first and second leg portions (31,32) in the first folded position and the wing 34 (more specifically, for receiving them on it).

### [First side wall 52]

The first side wall 52 is a wall that sections the connection hub 50H in the fixing member 5 into the first space 53 and the second space 59 together with the intermediate wall 57 and the second side wall 63 (the first side wall 52 sections it in the major length or longitudinal direction, and the intermediate wall 57 and the second side wall section it in the minor length or shorter direction).
A set of the first side walls 52 are formed (extend) as opposed to each other in the longitudinal direction of the horizontal axial line HL (that is, a pair of them is formed). Further, a pair of the first side walls 52 and 52 is formed on each of the first side portion S1 side and the second side portion S2 side.
Connecting the above pair of the first side walls 52 and 52, the second side wall 63 is formed on the proximal end PE side.
As shown in Fig. 7, further, a pair of the first side walls 52 and 52 has a pair of second latching portions 56 formed (protruded) on walls opposed to each other, the second latching portions 56 being opposed to each other. Each second latching portion 56 is formed in the form of a so-called "wedge", so that they can latch onto (be engaged with) the first latching portions 55.

### [Intermediate wall 57]

The intermediate wall 57 is a wall that sections the connection hub 50H of the fixing member 5 into the first space 53 and the second space 59 together with a pair of the first side walls 52 and 52 which extend in the longitudinal (elongated) direction. The intermediate wall 57 is formed between a pair of the first side walls 52 and 52, extending from the first side portion S1 side to the second side portion S2 side.
Further, the intermediate wall 57 is formed (protruded) somewhere on (nearly in the center position of) a pair of the first side walls 52 and 52, extending from the first side portion S1 side to the second side portion S2 side.
The intermediate wall 57 has the fixing member third latching portion 58 (to be sometimes abbreviated as "first latching portion 58" hereinafter) protruded on the distal end side and has the inclined wall 60 formed (protruded) on the proximal end side as shown in Fig. 10.

The above third latching portion 58 is a member that latches onto the groove 43 of slide member 42 of connection hub 40H of the shield member 3. The above third latching portion 58 latching onto the groove 43 of the connection hub 40H prevents the connection hub 40 from being pulled out of the connection hub 50H of the fixing member 5.
The third latching portion 58 is formed in the form of a so-called "rib". The latching portion 58 may be formed in any form so long as it can intrude into, and latch onto the groove 43 of the slide member 42 and thus formed of joint of latching portion 58 can prevent the connection hub 40 of the shield member 3 from being pulled out of the connection hub 50H of the fixing member 5.

### [Slide wall 60 (inclined wall 60)]

The slide wall 60 (to be sometimes referred to as "inclined wall 60" hereinafter) is a member that comes in contact with the inclined surface of inclined wall 45 of slide member 42 of connection hub 40H of the shield member 3. The inclined wall 60 has an inclined surface that goes down toward the proximal end PD side. The above inclined surface has a plurality of step portions in a stair-like form, as shown in Figs. 10, 15, and 17.
When the first and second leg portions (31,32) extend from the first folded position to the second extended position, the inclined wall 60 cooperatively therewith moves (or performs a so-called sliding) from the proximal end PE side to the distal end DE side, and while this sliding, the inclined wall 45 of slide member 42 of connection hub 40H of the shield member 3 is kept in contact with the inclined wall 60.
This constitution promotes the pulling of the distal end side wall 43DW of slide member 42 of the connection hub 40H into the space 40S of the connection hub 40H.

### [Second side wall 63]

The second side wall 63 is a wall that sections the connection hub 50H of the fixing member 5 into the first space 53 and the second space 59 together with the intermediate wall 57 and a pair of the first side walls 52 and 52.

### [First upper wall 61]

When the first and second leg portions (31,32) are in the first folded position, as shown in Figs. 7-9, the second latching portion 46 of the connection hub 40H is latched on the first upper wall 61 and it if inhibited or prevented from moving in the direction of an upper portion U as shown in Fig. 16.
When the first and second leg portions (31,32) finish extending from the first folded position to the second extended position, the second latching portion 46 of the shield member 3 latched and kept fixed by the upper portion (top surface) of first upper wall 61 of the fixing member 5 and works as a return-prevention hook (see Fig. 21). Further, the upper portion of third latching portion 47 of connection hub 40H of the shield member is latched and kept fixed by the lower portion (bottom surface) of the first upper wall 61 and works as a stopper, and it prevents the connection hub 40H of the shield member 3 from being pulled out of the connection hub 50H of the fixing member 5.

### [Second upper wall 62]

When the first and second leg portions (31,32) are in the first folded position, the second upper wall 62 as shown in Figs. 7-9, is for placing the first and second leg portions (31,32), the shield hub 35 and the wing 34 thereon, and for easily and stably placing them on it, it has a curved surface 63C formed as shown in Fig. 10.

### [Assembling to first state: assembling of shield member 3 and fixing member 5]

The process of assembling is explained, wherein shield member 3 is connected to fixing member 5 via its connection hub 40H, and shield hub 35 of shield member 3 is placed on fixing member 5, thereby brings it to the "first state".
<1> As shown in Fig. 1, the needle hub 22 is inserted into (the cylindrical space portion 35S of) the Shield hub 35 of the shield member 3 and fixed therein.
<2> Part of connection hub 40H of the shield member 3 is inserted into the first space 53 of connection hub 50H of the fixing member 5 from above and connected thereto.
<3> The cover 51 of the fixing member 5 is turned to the proximal end PE side at approximately 90° and closed.
In this case, the first latching portion 55 of fixing member of the cover 51 and the second latching portion 56 of the first side wall 52 are latched and kept fixed (whereby the cover 51 is fixed in a closed state).
As shown in Fig. 15 or Fig. 17, the wall surface of the first latching portion 48 of the connection hub 40H which is formed on the distal end DE side is brought into contact with a corresponding inside wall surface of the cover 51. On the other hand, the wall of the cover 51 which is on the proximal end PE side is brought into contact with the wall of the connection hub 40H which is on the distal end DE side.
The third latching portion 58 of the intermediate wall 57 is latched and kept fixed by the groove 43 of the slide member 42, and it works as a stopper for preventing the connection hub 40H of the shield member 3 from being pulled out of the connection hub 50H of the fixing member 5.
Further, the inclined surface of inclined wall (slide wall) 45 of the slide member 42 is brought into contact with that surface of inclined wall 60 of the fixing member 5 which is on the downward portion D side.

<4> The first and second leg portions (31,32) of the shield member 3 are folded, and the distal end 23 side of the needle 21 is inserted into the space 40S of the connection hub 40H.
<5> The first and second leg portions (31,32) are folded as illustrated in Fig. 12 or 15, and they are housed in the second space 59 shown in Fig. 7 together with the lower portion of the shield hub 35. [That is, they are placed on the space 59. The first and second leg portions (31,32) are placed in a position in an upper portion of the second space 59].
   The pair of the second latching portion 46 of the shield member 3 is latched and kept fixed by the downward portion of the first upper wall 61 as shown in Fig. 16.
<6> The needle cover 6 is attached on the distal end 23 side of the needle 21 to protect it.

### [Protection (shielding) of distal end 23 of needle 21]

### <1> "Transition start state" (Fig. 15)

When the needle 21 is lifted up to the upper portion U side of the vertical axial line VL, the first and second leg portions (31,32) start cooperatively therewith extending to the upper portion U side of the vertical axial line VL.
Further, cooperatively therewith, the inclined surface of inclined wall 45 of the slide member 42 starts to move to the distal end DE side along that inclined surface of inclined wall 60 of connection hub 50H of the fixing member 5 which is on the downward portion D side. (It starts a so-called "sliding".) The second latching portion 46 of the shield member 3 also starts moving toward the upper portion U (Fig. 16).

### <2> <Transition end state>

The first and second leg portions (31,32) are about to extend fully toward the upper portion U side of the vertical axial line VL.
The second latching portion 46 of the connection hub 40H of the shield member 3 is about to go over the first upper wall 61 of the fixing member 5 through the upper opening portion 50OU.
The distal end side wall 43DW of the slide member 42 is pulled into the space 40S of the connection hub 40H to shift in position so as to close or block the cross section of the space 40S.

### <3> (Second state)(Fig. 19)

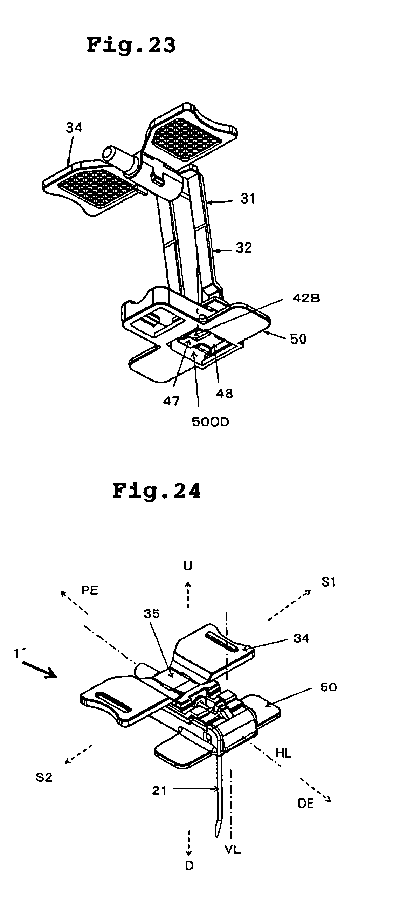

The first and second leg portions (31,32) completely extend to the upper portion U side of the vertical axial line VL.
The second latching portion 46 of the shield member 3 is latched and kept fixed by the upper portion (top surface) of first upper wall 61 of the fixing member 5 (see Fig. 21). The above second latching portion 46 constitutes a return-prevention hook. Further, the upper portion of third latching portion 47 of the shield member 3 is latched and kept fixed by the lower portion (bottom surface) of the first upper wall 61, and it constitutes a stopper and can prevent the connection hub 40H of the shield member 3 from being pulled out of the connection hub 50H of the fixing member 5 (see Fig. 21).
The cross section of space 40S of connection hub 40H of the shield member 3 is blocked by the distal end side wall 43DW of the slide member 42. Further, the cross section of opening portion 400 (or vicinities of the opening portion 400) of the connection hub 40H is blocked by the downward portion wall 42B (Fig. 20).
The distal end 23 of the needle 21 is contained or accommodated in the space 40S to be protected (shielded). This prevents the distal end 23 of the needle 21 from moving to the downward portion U lower than the distal end side wall 43DW to be exposed to an outside (see Figs. 22 and 23).

Embodiments including the attaching of the apparatus to a patient, the injection of a medicinal reagent solution, the removal of the apparatus from the patient and the disposal of the apparatus will be further explained.

### [Attaching of the apparatus to patient and injection of a medicinal reagent solution]

<1> In the first state (folded state), the top of the needle is protected by the needle cover 6 attached thereto. When in use, the needle cover 6 is removed to expose the distal end 23 of the needle 21.
<2> The distal end 23 of the needle 21 is moved downward in the direction of downward portion D of the vertical axial line VL and punctured in a port (not shown) implanted in a patient. The flat contact surface plate 50 of the fixing member 5 is fixed to the surface of the body of the patient with a tape, or the like.
<3> A medicinal reagent solution containing anticancer drug or the like is injected from the medicinal reagent solution injection tube T connected to the proximal end PE side of the needle hub 22 through the distal end 23 of the needle 21 and the port (not shown). In this first state, transfusion of the reagent solution, or the like is conducted. (Thus, the first state shown in Fig. 12, etc., refers to a state wherein, for example, transfusion, or the like is carried out.)

### [Removal of the apparatus from patient to disposal]

After completion of the injection of the medicinal reagent solution, a worker (doctor or nurse), while letting one hand touch the skin of the patient, takes a grip on the wing portions 34 with the other hand and lifts it up toward the upper portion U (Fig. 14).
In this manner, the distal end 23 of the needle 21 is pulled out from the port (not shown), and the first and second leg portions (31,32) are extended. While the first and second leg portions (31,32) are extending, the distal end 23 of the needle 21 is taken into the space 40S of connection hub 40H of the shield member, and further, the distal end side wall 43DW of the above connection hub 40H shifts in position so as to block the space 40S of the connection hub 40H.
In this constitution, the distal end side wall 43DW of the connection hub 40H can prevent the distal end 23 of the needle 21 from again moving in the direction of the downward portion D lower than the distal end side wall 43DW. When the first and second leg portions (31,32) are completely extended, the needle safety apparatus 1 is fixed to a home position, and the distal end 23 of the needle 21 can be completely protected (shielded) by the connection hub 40H.
The worker (doctor or nurse) can dispose of the needle 21 without suffering any fear of an incidental needle puncture caused by an accidental exposure of the needle.

### [Other embodiments of the invention]

The safety apparatus 1 for medical needles according to the invention is not limited to the embodiment shown in Figs. 1 to 23, Figs. 50-51, and Figs. 54-55. The embodiment of the following Example will be included in the technical scope of this invention. With regard to the other Example, portions (members) therefor will be explained below in detail one by one.
That is, there may be employed any constitution in which, when the leg portions (31,32) extend from the first folded position to the second extended position, at least the distal end side wall 43DW (or part of the distal end side wall 43DW) of the slide member 42 is pulled into the space 40S of the connection hub 40H, whereby the distal end side wall 43DW (or part of the distal end side wall 43DW) of the slide member 42 can block the cross section of space 40S of the shield member connection hub 40H, so that the distal end 23 of the needle 21 can be prevented from moving to the downward portion D lower than the wall 43DW of the slide member 42 and that the distal end 23 of the needle 21 can be prevented from being exposed to an outside of the hub.

### [Leg portions (31,32)]

There may be employed any embodiment in which the leg portions (31,32) can extend from the first folded position to the second extended position, and when they are in the second extended position, the distal end 23 of the needle 21 and the distal end side wall 43DW of the slide member 42 of the shield members are pulled into the connection hub 40H to be protected (shielded).
Figs. 1 to 3 show an embodiment of leg portions (31,32) having a so-called "nearly plate form", while they may have any form so long as they accomplish the above function, and they may be any embodiment having a so-called "a string form, a rod form, a band form, a chain form" or the like.

### [Shield hub 35]

The shield hub 35 may be employed any embodiment in which the needle hub 22 can be fixed and when the leg portions (31,32) extend from the first folded position to the second extended position, it can lift up the needle 21 in the direction of the upper portion U cooperatively with the leg portions (31,32).

### [Connection hub 40H of shield member]

In illustrations in Figs. 2 and 3, the central portion of the connection hub 40H is formed in a so-called "angularly cylindrical form" (a cross section has the form of a rectangle", while it shall not be limited thereto. It may be any embodiment having a space 40S of a cylindrical form in cross section, an elliptical form in cross section, etc., so long as the distal end 23 of the needle 21 and the distal end wall 43DW of the slide member 42 can be pulled into it to be protected (shielded).
Further, it may be any embodiment so long as it has the above slide member 42 and the first latching portion 48, the second latching portion 46 and the third latching portion 47 of the shield member 3. Further, it may be any embodiment so long as it can perform the functions of each portion or member.
In an embodiment illustrated in Fig. 20, the distal end wall 43DW of the slide member 42 blocks the cross section of the space 40S in an inclined position, while it can block the same in a horizontal position.
In the second extended position of the leg portions (31,32), the position of distal end wall 43DW of the slide member 42 may be in an inclined position or in a horizontal position.
In an embodiment illustrated in Fig. 20, the downward portion wall 42B of the slide member 42 blocks the cross section of the opening portion 400 (or vicinities of the opening portion 400) in a downward portion of the connection hub 40H in an inclined position, while it can block the same in a horizontal position.
In the second extended position of the leg portions (31,32), the position of the downward portion wall 42B may be in an inclined position or in a horizontal position.

### [Slide member 42]

In an embodiment illustrated in Figs. 5 and 6, the slide member 42 is formed in the form of a so-called "nearly the letter L", while it may be any member having a so-called "wall" or (wall portion) that can work cooperatively with the leg portions (31,32) when they extend from the first folded position to the second extended position and that can block the space 40S of connection hub 40H of the shield member.
Further, it is ensured owing to the "inclined wall 45" (to be also referred to as "slide wall 45") that the slide member 42 performs a so-called "sliding" when the leg portions (31,32) extend from the first folded position to the second extended position as illustrated in Figs. 5 and 6. (More specifically, the sliding member 42 slides while the inclined wall 45 of the slide member 42 is being in contact with the inclined wall 60 of the fixing member 5.)
The slide member 45 may be any embodiment so long as it can slide as described above. For example, it may be formed in the form of a "curved wall", etc., in place of the "inclined wall 45". In this case, the connection hub 50H of the fixing member 5 may be also formed in the form of a "curved wall" in place of the "inclined wall 60". These "curved walls" are also included in the "slide wall".

Further, as illustrated in Figs. 5 and 6, the slide member 42 is so formed as to have a constitution in which the third latching portion 58 of connection hub 50H of the fixing member 5 is latched and kept fixed by the "groove 43" to prevent the connection hub 40H of the shield member from being pulled out of the connection hub 50H of the fixing member 5. It may be any embodiment so long as the connection hub 40H cannot be pulled out of the connection hub 50H.
For example, "a rib" may be formed in place of the "groove 43". In this case, in concert therewith, a "groove" in place of the third latching portion 58 (rib) may be formed in the connection hub 50H of the fixing member 5 so that the rib may intrude in the "groove" and may be latched and kept fixed.

### [First latching portion 48, second latching portion 46 and third latching portion 47 of shield member 3 (connection hub 40H)]

The first latching portion 48, second latching portion 46 and third latching portion 47 of the shield member may have any constitution so long as they work to keep the connection hub 40H from being pulled out of the connection hub 50H of the fixing member 5 as described above when the first and second leg portions (31,32) extend from the first folded position to the second extended position.
The above first latching portion 48 may have the form of a so-called "(hook) nail" or "a sword guard (flange)" in place of the form of "protrusion" (a nearly trapezoidal form) as illustrated in Fig. 4.
Further, the second latching portion 46 may have the form of a so-called "a sword guard (flange)" or the form of "protrusion" in place of a so-called "(hook) nail" as illustrated in Fig. 4.
Further, the third latching portion 47 may have the form of a "(hook) nail" or the form of "protrusion" in place of a so-called "a sword guard (flange)" as illustrated in Fig. 4.

### [Connection hub 50H of fixing member 5]

The connection hub 50H of the fixing member 5 may have any constitution so long as it works to keep the connection hub 40H of the shield member 3 from being pulled out as described above.
More specifically, the first space 53 may have any constitution so long as it can contain the connection hub 40H and fix the same therein.
The second space 59 may have any constitution so long as it can contain or accommodate the cylindrical shield hub 35 of the shield member 3, the first and second leg portions (31,32) in the first folded position and the wings 34 therein (can have these thereon).
The first side wall 52, the intermediate wall 57 and the second side wall 63 may have any constitution so long as they can section the connection hub 50H into the first space 53 and the second space 59 as described above.
The cover 51 may have any constitution so long as it works to fix the connection hub 40H of the shield member 3 as described above.
The first latching portion 55 and second latching portion 56 of the fixing member 5 may have any constitution so long as they can be latched and kept fixed by each other so that the cover 51 can close the opening surface in the DE direction of connection hub 50H of the fixing member 5 as shown in Figs. 9 and 11.
The third latching portion 58 and the inclined wall 60 of the fixing member 5 work as described above.

Since the shield member 3 and the fixing member 5 for constituting the safety apparatus 1 for medical needles are preferably produced by integral molding, the production is easy, and they can be produced at a low cost as compared with the invention described in Patent Document 1.
In the shield member 3, preferably, the shield hub 35, the leg portions (31,32) and the connection hub 40H are preferably formed from the same material by integral molding. In the connection hub 40H, preferably, the second latching portion 46 and the third latching portion 47 are formed from the same material by integral molding in each of the first side portion S1 side and the second side portion S2 side.
In the fixing member 5, preferably, the connection hub 50H and the flat contact surface plate 50 are formed from the same material by integral molding.

In the connection hub 50H, preferably, a plurality of the walls (52, 57 and 63), the cover 51, the third latching portion 58 and the slide wall (inclined wall) 60 are formed from the same material by integral molding.
The material for the shield member 3 and the fixing member 5 is preferably a thermoplastic synthetic resin that can be used for injection molding, extrusion molding, blow molding and cast molding. Examples of the material include PE (polyethylene), PP (polypropylene), PB (polybutylene), PS (polystyrene), PVC (polyvinyl chloride), PVdC (polyvinylidene chloride), PMMA (polymethyl methacrylate), PMA (polymethyl acrylate), PC (polycarbonate), PAm (Polyamide) and ETFE copolymers, while the material shall not be limited thereto. For example, they can be formed from a thermosetting resin.

### [Safety apparatus 1' for medical needles] (second invention (second embodiment))

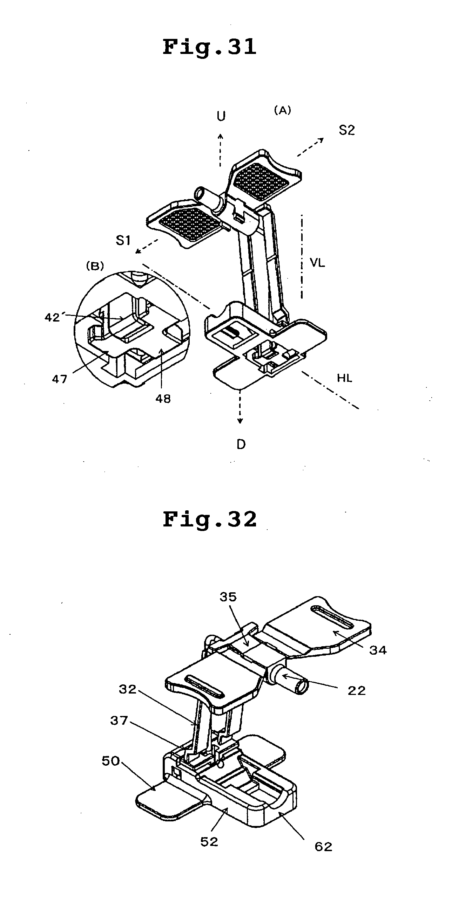

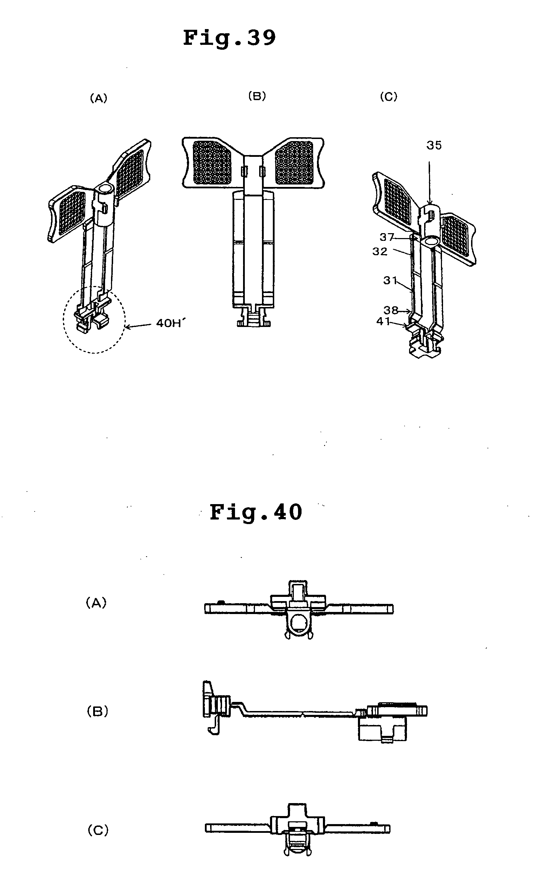

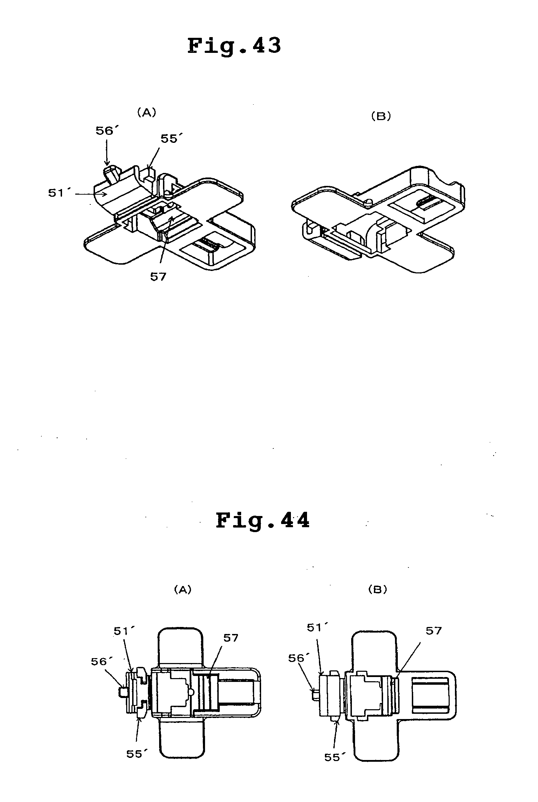

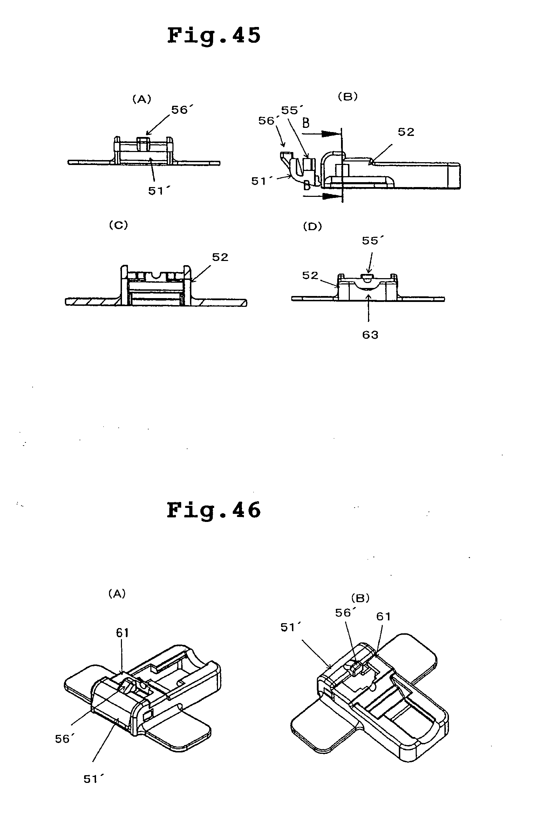

The safety apparatus for medical needles according to the second invention (second embodiment) will be explained below with reference to Figs. 24 to 49 and Figs. 52-53.
In the explanation of the safety apparatus 1' for medical needles according to the second invention (embodiment), the members which are common to, or substantially the same as, those of the safety apparatus 1 for medical needles according to the first invention are shown by the same reference numerals for avoiding complications of the numerals. In the safety apparatus 1' for medical needles according to the second invention (embodiment), further, parts which are different from, but have substantially the same or corresponding functions as/to, the counterparts in the safety apparatus 1 for medical needles according to the first invention are shown by the same numerals to which "'" is added for avoiding complications of the numerals.

### [Shield member 3']

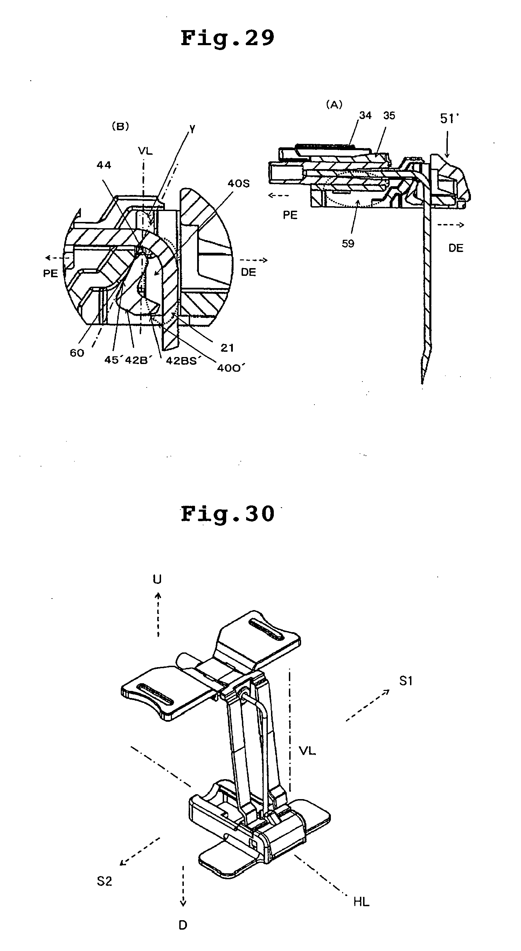

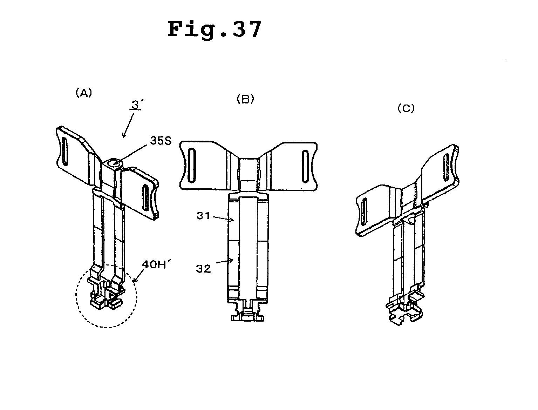

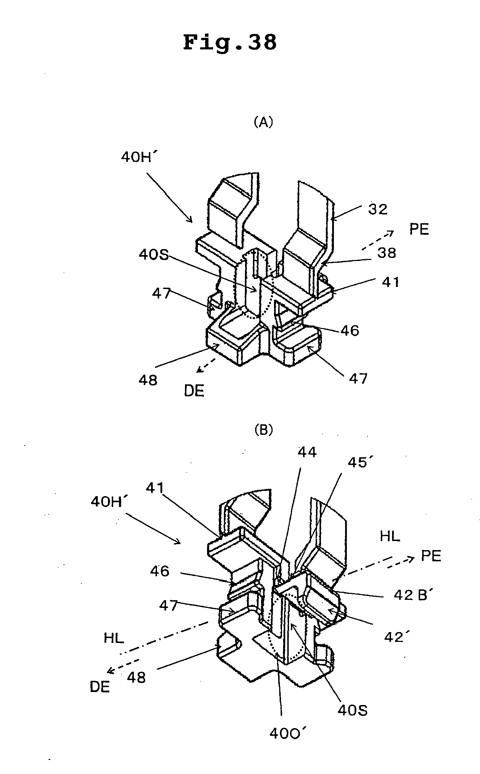

The shield member 3' of the safety apparatus 1' for medical needles according to the second invention (embodiment) has a form (shape and structure) illustrated in Figs. 37 to 40.
When the safety apparatus 1' for medical needles according to the second invention (embodiment) is compared with the safety apparatus 1 for medical needles according to the first invention (embodiment), the shield member connection hub 40H' (to be sometimes abbreviated as "connection hub 40H'" hereinafter) in the shield member 3' is partially different in form. The shield member 3' (of the second invention (embodiment))] differs in that the slide member 42' (of the connection hub 40H') (having a slide wall 45' and a downward portion wall 42B') is formed nearly in the form of the letter L as illustrated in Figs. 29 and 35 and in particular in Fig. 38 in place of the slide member 42 [having the distal end side wall 43DW, slide wall 45 (to be also referred to as "inclined wall 45"), the downward portion wall 42B and the groove 43] (of the first invention (embodiment)).

More specifically, the shield member 3' (of the second invention) uses one (a) slide wall 45' as shown in Fig. 38(B) in place of the distal end side wall 43DW, the groove 43 and the inclined wall 45 (a pair of these is formed on the first side portion S1 side and the second side portion S2 side) formed in the slide member 42 (of the first invention).
(b) It uses a downward portion wall 42B' formed in a downward portion of one slide wall 45' in place of the downward portion wall 42B formed in a downward portion of the distal end side wall 43DW, groove 43 and inclined wall 45 of the slide member 42 (of the first invention(embodiment)).

### [(Second) slide member 42']

Further, the slide member 42' of the second invention (embodiment) will be explained in detail. The slide member 42' is sometimes designated as second slide member 42'. The slide member 42' has a slide wall 45' and a downward portion wall 42B', and these are integrally formed nearly in the form of the letter L.
The slide wall 45' has that one end portion on the distal end DE side which is connected to a hinge 44 as illustrated in Fig. 38(B). The other end portion on the proximal end PE side is connected to the downward portion wall 42B' by integral molding) as illustrated in Fig. 38(B). The slide member 42' is connected to the proximal end PE side in the direction of an upper portion U of the connection hub 40H through a hinge 44.

In this constitution, when the first and second leg portions (31,32) extend from the first folded position to the second extended position, the slide member 42' moves cooperatively therewith. That is, when the first and second leg portions (31,32) extend from the first folded position to the second extended position, the slide member 42' performs a "swing" from a position in Fig. 29 (the first position) to a position in Fig. 35 (the second position) with the hinge 44 as a start point to shift in position such that the downward portion wall 42B' blocks the cross section of the opening portion 40O' of bottom portion (in the direction of a downward portion D) of the connection hub 40H'. This is as explained in detail in the first invention (embodiment).

Still more specifically, the "first position" of the slide member 42' means a position in which the downward portion wall 42B' is in a position in Fig. 29 and keeps blocking no cross section of opening portion 40O' of bottom portion (in the direction of a downward portion D) of the connection hub 40H'.
The "second position" of the slide member 42' means a position in which the downward portion wall 42B' is in a position in Fig. 35 and blocks the cross section of opening portion 40O' of bottom portion (in the direction of a downward portion D) of the connection hub 40H'.

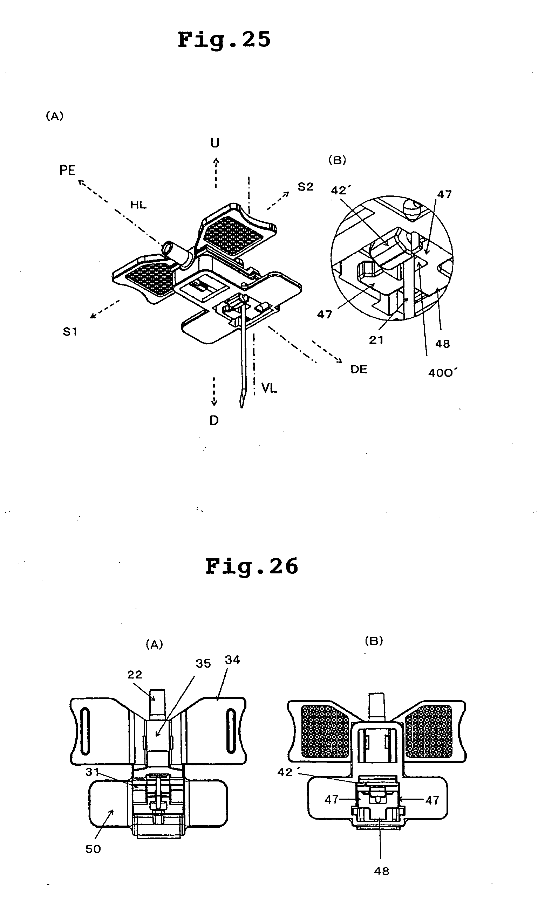

The slide wall 45' is a member which comes in contact with the slide wall 60 (inclined wall 60) of fixing member connection hub 50H' of the fixing member 5'. (The slide wall 45' slides while maintaining this contact.)
The fixing member connection hub 50H' (to be sometimes abbreviated as "connection hub 50H'" hereinafter) of the fixing member 5' in the second invention (embodiment) will be described in detail later.
The slide wall 45' is formed substantially in the form of a "nearly horizontal" and "board like plate" as illustrated in Fig. 29, 35 and 38.
In the slide wall 45', further, notches like concavo-convex shapes or caterpillar form are made in an end portion on the proximal end PE side (note: they are shown as "lateral lines" in Fig. 25). The above notches in the form of concavo-convex shapes or caterpillar form are provided to a proper degree, to ensure that the slide wall 45' is slidable easily and moderately relative to the slide wall 60 (inclined wall 60) of the fixing member 5' and also ensure that it does not move at once or is too immovable.

When the first and second leg portions (31,32) extend from the first folded position to the second extended position, the downward portion wall 42B' is a member that cooperatively therewith closes the opening portion 40O' of the connection hub 40H'.
In the downward portion wall 42B', further, a cut portion 42BS is formed "not nearly horizontally" but in the direction of a downward portion D and on the distal end DE side as illustrated in Figs. 29, 35 and 38. (Further, notches like concavoconvex shapes or caterpillars are formed on an end portion on the proximal end PE side or the slide wall 60 (inclined wall 60) side (note: they are shown as lateral lines in Fig. 25 as described above).

A case which employs a combination of the slide member 42' and the slide wall 60 (inclined wall 60) of the fixing member 5' will be explained below.
Before assembling, the slide wall 45' is nearly in parallel with the horizontal axial line HL as illustrated in Fig. 38(B).
In the first state after assembling ("the first position" of the slide member 42'), the slide wall 45' is arranged such that it intersects the vertical axial line VL at a sharp angle γ as is illustrated in Fig. 29. In other words, it is slantedly arranged as it goes up from the proximal end PE side to the distal end DE side.
In the second state after assembling ("the second position" of the slide member 42'), the slide wall 45' is nearly in parallel with the vertical axial line VL as illustrated in Fig. 35.

When the first and second leg portions (31,32) extend from the first folded position to the second extended position, the slide wall 45' cooperatively therewith moves (or performs a so-called sliding) from the proximal end PE side to the distal end DE side in which it is kept in contact with the inclined surface of the above inclined wall 60. In this constitution, when the first and second leg portions (31,32) extend from the first folded position to the second extended position, the downward portion wall 42B' cooperatively therewith promotes or facilitates the action to close the opening portion 40O' of connection hub 40H of the shield member.

When the slide member 42' is in the first state [when the first and second leg portions (31, 32) are in the first folded position], the downward portion wall 42B' of the slide member 42' does not block any cross section of opening portion 40O' of the connection hub 40H' (that is, the needle sticks out of the opening portion 40O' and extends downward).
In other words, in the first state, the needle 21 is inserted in the space 40S of the connection hub 40H' (see Fig. 29).
In the second state (when the first and second leg portions (31,32) are in the second extended position), the downward portion wall 42B' of the slide member 42' closes the opening portion 40O' of the connection hub 40H' (see Fig. 35).
By this constitution, the downward portion wall 42B' of the slide member 42' blocks the opening portion 400' of connection hub 40H' of the shield member and prevents the distal end 23 of the needle 21 from (again) moving a downward portion D lower than the downward portion wall 42B' of the slide member 42'. This constitution can prevent the distal end 23 of the needle 21 from being exposed from the opening portion 40O' of the above connection hub 40H' (see Fig. 35).

### [Fixing member 5']

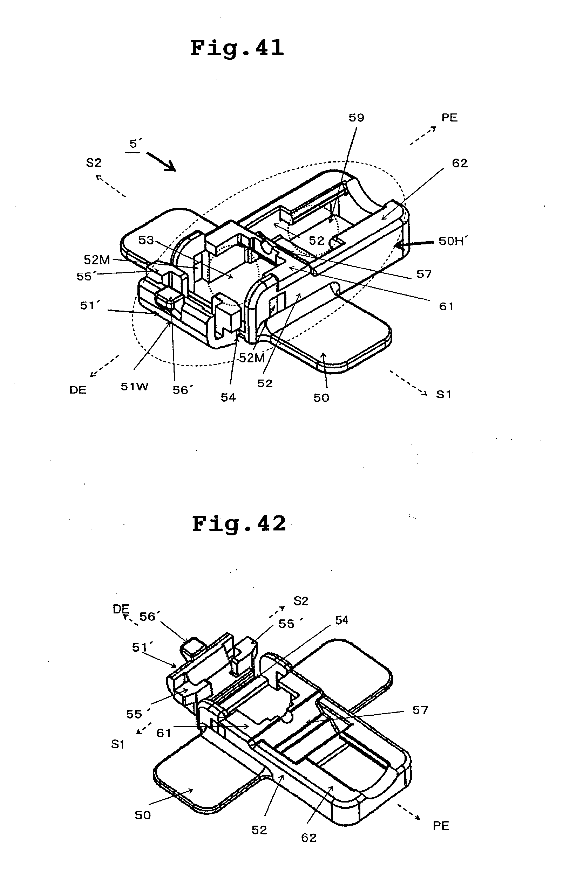

The fixing member 5' in the safety apparatus 1' for medical needles according to the second invention (embodiment) has a form (shape and structure) illustrated in Figs. 41 to 49.
When the safety apparatus 1' for medical needles according to the second invention (embodiment) is compared with the safety apparatus 1 for medical needles according to the first invention (embodiment), the connection hub 50H' of the fixing member 5' and the cover 51' are different in form.
When the connection hub 50H' of the safety apparatus 1' for medical needles according to the second invention (embodiment)is compared with the connection hub 50H of the safety apparatus 1 for medical needles according to the first invention (embodiment), (a) the second latching portion 56 formed inside the first side wall 52 (in the first invention (embodiment)) is omitted (in the second invention (embodiment)).

(b) Further, that third latching portion 58 of the fixing member which is formed on the distal end DE side of the intermediate wall 57 (in the first invention (embodiment)) is omitted.
   Further, when the cover 51' of the safety apparatus 1' for medical needles according to the second invention (embodiment)is compared with the cover of the safety apparatus 1 for medical needles according to the first invention (embodiment), (c) the form of the first latching portion 55 (in the first invention (embodiment)) is modified to a form of the first latching portion 55' (in the second invention (embodiment)) as shown in Fig. 41.
(d) (In the second invention (embodiment)) A novel fixing member second latching portion 56' (to be sometimes abbreviated as "novel second latching portion 56'" hereinafter) is formed in the direction of an upper portion and on the distal end DE side as illustrated in Fig. 41. The novel second latching portion 56' works as a so-called "pressing member" or "fixing member" for pressing the closed cover 51'.

The fixing member first latching portion 55' (to be sometimes abbreviated as "first latching portion 55'" hereinafter) (of the second invention (embodiment)) will be explained in detail bellow.
In the first latching portion 55 (of the first invention), a pair of them is protruded toward the proximal end PE side from the distal end DE side of the cover 51 as explained already (for example, see Fig. 7).
Further, the first latching portion 55 (in the first invention) is formed in the form of a so-called "wedge" such that the first latching portion 55 and the second latching portion 56 formed inside the first side wall 52 latch onto each other. In contrast, in the first latching portion 55' (in the second invention), a pair of them is protruded on the first side portion S1 side and the second side portion S2 side such that they stand upward nearly perpendicularly toward an upper portion U from a bottom wall (downward portion D) of the cover 51' as illustrated in Figs. 41 to 49.

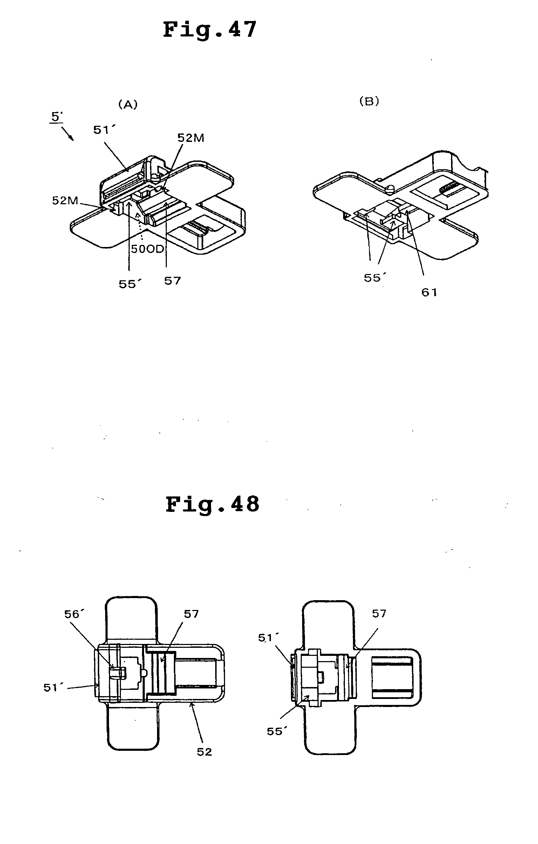

The cross section of the first latching portion 55' in the direction of the horizontal axial line HL (flat surface viewed from the direction of an upper portion U) has the form of a so-called the letter L. Further, it is also similar to the form of "a crab claw" or "a beetle's antenna".
The cover 51' of the fixing member is formed so as to ensure that it turns through a hinge 54 toward the proximal end PE side at approximately 90° and thereby the first latching portion 55' can latch onto the downward portion D wall of the first upper wall 61 (in the second invention (embodiment), the second latching portion 56 is absent or non-existant, quite different from the fixing member 5 in the first invention (embodiment)) as illustrated in Figs. 41 to 49 (see Fig. 47(B)).

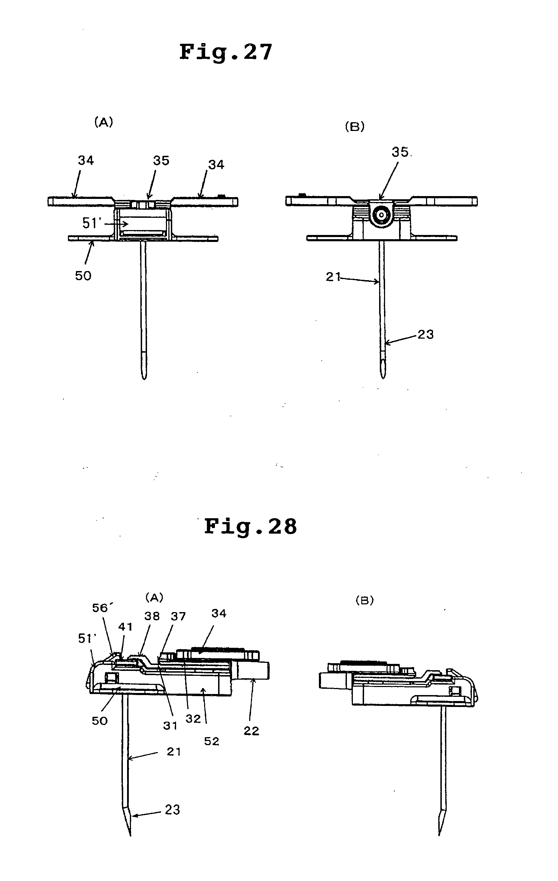

The novel second latching portion 56' is formed to further enhance the connection and fixing of the connection hub 40H' of the shield member 3' after the connection hub 40H' is inserted into, and connected to, the first space 53 of the connection hub 50H' of the fixing member 5'. The novel second latching portion 56' is formed in the form of a so-called "a hook nail" as illustrated in Figs. 41 to 49.
Further, the novel latching portion 56' has a function to prevent the distal end 23 of the needle 21 from being exposed when the second state is brought up.
When the cover 51' is turned toward the proximal end PE side at 90° through the hinge 54 to latch onto the downward portion D wall of the first upper wall 61 as described above, the novel latching portion 56' concurrently presses the upper portion connection portion 41 of the connection hub 40H' from the direction of an upper portion U as illustrated for example in Fig. 28. This constitution can prevent the cover 51 that is once closed from turning in the direction of the opposite side to open.

### [Assembling to first state: Assembling of shield member 3' and fixing member 5']

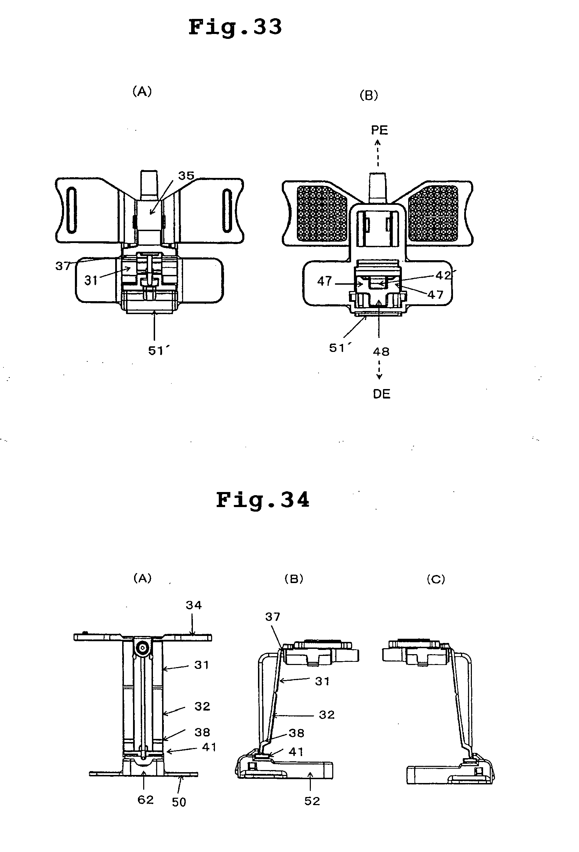

Following the same manner as is explained with respect to the first invention (embodiment), the process of assembling is performed, wherein shield member 3' is connected to fixing member 5' via its connection hub 40H', and shield hub 35' of shield member 3' is placed on fixing member 5', thereby brings it to the "first state".
<1> As shown in Fig. 36, the needle hub 22 is inserted into the shield hub 35 of the shield member 3' and fixed therein.
<2> The connection hub 40H' of the shield member 3' is inserted into the first space 53 of connection hub 50H' of the fixing member 5' and connected thereto.
<3> The cover 51' is turned toward the proximal end PE side at approximately 90° and closed.
   In this case, as shown in Fig.41, the first latching portion 55' of the cover 51' and the downward portion D wall of first upper wall 61 of the connection hub 50H' are latched and kept fixed by each other.
   That is, the distal end DE side wall surface of first latching portion 48 of connection hub 40H' of the shield member is brought into contact with the wall surface of downward portion D side of distal end DE side of connection hub 50H' of the fixing member as illustrated in Fig. 33. That is, the side wall of proximal end PE of the cover 51' is brought into contact with the distal end DE side wall of the connection hub 40H' as illustrated in Fig. 29(A).
<4> At the same time, the novel second latching portion 56' presses the upper portion connection portion 41 of the connection hub 40H' from the direction of an upper portion U as illustrated, for example, in Fig. 28, etc. Thus, the cover 51' that is once closed does not in any case turn in the opposite direction to open. Further, the slide wall 45' of the slide member 42' comes in contact with the downward portion D side inclined surface of slide wall (inclined wall) of the fixing member 5' as illustrated, for example, in Fig. 29.

<5> The first and second leg portions (31,32) of the shield member 3' shown in Fig. 36 are folded, and the distal end 23 side of the needle 21 in Fig. 36 is inserted into the space 40S (see Fig. 38) of connection hub 40H' of the shield member.
<6> The first and second leg portions (31,32) are folded as illustrated, for example, in Fig. 29(A), and are contained or accommodated in the second space 59 (see Fig. 41) of the fixing member 5' together with the downward portion of the shield hub 35. [That is, they are placed on the space 59. The first and second leg portions (31,32) are placed in the position of upper portion of the second space 59.
   The second latching portion 46 of the shield member is latched and kept fixed by the downward portion of the first upper wall 61 (see Fig. 16 in the first invention (embodiment)).
<7> The needle cover 6 is fit onto the distal end 23 side of the needle 21 to protect it, as shown in Fig.36.

### [Protection (shielding) of distal end 23 of needle 21]

### <1> "Transition start state" (Fig. 24)

When the needle member 21 is lifted up to the upper portion U side of the vertical axial line VL from the first position shown in Fig. 24, the first and second leg portions (31,32) cooperatively therewith start to extend to the upper portion U side of the vertical axial line VL.
Further, cooperatively therewith, the slide wall 45' of the slide member 42' starts to move to the distal end DE side along that inclined surface of slide wall (inclined wall) 60 of connection hub 50H of the fixing member which is on a downward portion D side. (It starts to perform a so-called "sliding"). The second latching portion 46 of the shield member also starts to move in the direction of the upper portion U.

### <2> "Transition end state"

The first and second leg portions (31,32) are about to extend fully toward the upper portion U side of the vertical axial line VL.
The second latching portion 46 is about to go over the first upper wall 61 through the upper opening portion 50OU.
The downward portion wall 42B' of the slide member 42 is pulled into the space 40S of the connection hub 40H' to shift in position so as to close the opening portion 400'as shown in Fig.35.

### <3> (Second state)(Fig. 30)

The first and second leg portions (31,32) completely extend to the upper portion U side of the vertical axial line VL.
The second latching portion 46 of the shield member 3' is latched and kept fixed by the upper portion (top surface) of the first upper wall 61. The above second latching portion 46 constitutes a return-prevention hook. Further, the upper portion of third latching portion 47 is latched and kept fixed by the lower portion D (bottom surface) of the first upper wall 61, and it constitutes a stopper and can prevent the connection hub 40H' of the shield member 3 from being pulled out of the connection hub 50H' of the fixing member 5'. The embodiment of the above latching is basically similar to that shown in Fig. 21 in the first invention (embodiment)..
The cross section of opening portion 40O' (or vicinities of the opening portion 40O') of the connection hub 40H' is blocked by the downward portion wall 42B' of the slide member 42'. The distal end 23 of the needle 21 is contained or accommodated in the space 40S and protected (shielded). This prevents the distal end 23 of the needle 21 from moving to the downward portion U lower than the downward portion wall 42B' to be exposed to an outside (see Fig. 35 showing the second position).

Embodiments including attaching the apparatus to a patient, injecting a reagent solution, removing the apparatus from the patient and disposing of the apparatus will be further explained, while these are basically similar to those explained in the first invention (embodiment) and will be hence explained simply.

### [Attaching the apparatus to a patient and injecting a medicinal reagent solution]

<1> The needle cover 6 is removed to expose the distal end 23 of the needle 21.
<2> The distal end 23 of the needle 21 is moved downward in the direction of downward portion D of the vertical axial line VL and punctured in a port (not shown) implanted in a patient. The flat contact surface plate 50 of the fixing member 5 is fixed to the patient with a tape, or the like.
<3> A medicinal reagent solution containing anticancer drug or the like is injected from the medicinal reagent solution injection tube T connected to the proximal end PE side of the needle hub 22 through the distal end 23 of the needle 21 and the port (not shown).

### [Removal of the apparatus from patient to disposal]

After completion of the injection of the reagent solution, a worker (doctor or nurse), while letting one hand touch the skin of the patient, takes a grip on the wing portions 34 with the other hand and lifts it up to the upper portion U.
In this manner, the distal end 23 of the needle 21 is pulled out from the port (not shown), and the first and second leg portions (31,32) are extended. While the first and second leg portions (31,32) are extending, the distal end 23 of the needle 21 is taken into the space 40S of connection hub 40H', and further, the downward portion wall 42B' of the connection hub 40H' shifts in position so as to block the cross section of opening portion 40O' of the connection hub 40H'.
In this constitution, the downward portion wall 42B' of the connection hub 40H' can prevent the distal end 23 of the needle 21 from again moving in the direction of the downward portion D lower than the downward portion wall 42B'. When the first and second leg portions (31,32) are completely extended, the needle safety apparatus 1' is fixed to a home position, and the distal end 23 of the needle 21 can be completely protected (shielded) by the connection hub 40H'.
The worker (doctor or nurse) can dispose of the needle 21 in this manner without suffering from any concerns of an incidental needle sticking caused by an accidental exposure of the needle.

The safety apparatus 1' for medical needles according to this invention is not limited to the embodiment (or shape) shown in Figs. 24 to 49.
That is, safety apparatus 1' for medical needles is not limited to the above embodiment so long as it insures that when the leg portions (31,32) extend from the first folded position to the second extended position, the downward portion wall 42B' of the slide member 42' is pulled into the space 40S of the connection hub 40H', whereby the downward portion wall 42B' of the slide member 42' can block the cross section of opening portion 40O' of the connection hub 40H', so that the distal end 23 of the needle 21 can be prevented from moving to the downward portion D lower than the downward portion wall 42B' of the slide member 42' and that the distal end 23 of the needle 21 can be prevented from being exposed to an outside.

### [Leg portions (31,32)]

There may be employed any embodiment in which the leg portions (31,32) can extend from the first folded position to the second extended position, and when they are in the second extended position, the distal end 23 of the needle 21 and the downward portion wall 42B' of the shield member 42' can be pulled into the connection hub 40H' to be protected (shielded).
Figs. 24 to 26 illustrate an embodiment of the leg portions (31, 31) having a so-called "nearly plate form", while they shall not be limited thereto and may be any embodiment having a so-called "a string form, a rod form, a band form, a chain form" or the like.

### [Shield hub 35]

The shield hub 35 shall not be limited to the embodiment that illustrated above so long as it is an embodiment in which the needle hub 22 can be fixed and when the leg portions (31, 32) extend from the first folded position to the second extended position, it can lift up the needle 21 in the direction of the upper portion U cooperatively with the leg portions (31,32).

### [Connection hub 40H']

In illustrations in Figs. 37 to 40, the central portion of the connection hub 40H' is formed in a so-called "nearly angularly cylindrical form" (a rectangular form in cross section), while it may be any other embodiment that is already described with regard to the first invention (embodiment) so long as the distal end 23 of the needle 21 and the downward portion wall 42B' of the slide member 42' can be pulled into it to be protected (shielded).
Further, it may be any embodiment so long as it has the above slide member 42' and the first latching portion 48, the second latching portion 46 and the third latching portion 47 of the shield member 3'.
In an embodiment illustrated in Fig. 35, the downward portion wall 42B' of the slide member 42' blocks the cross section of the opening portion 40O' nearly in a horizontal position, while it can block the same nearly in an inclined position.
In the second extended position of the leg portions (31,32), the position of the downward portion wall 42B' may be in an inclined position or in a horizontal position.

### [Slide member 42']

In an embodiment illustrated in Figs. 29, 35 and 38, the slide member 42' is formed nearly in the form of the letter L (having the slide wall 45' and the downward portion 42B'), while it may be any other embodiment having a so-called "wall" that can work cooperatively with the leg portions (31,32) when they extend from the first folded position to the second extended position and that can be pulled into the space 40S of the connection hub 40' to block the cross section of opening portion 40O' of the above connection hub 40H'.

Further, it is ensured, owing to the "inclined wall 45'", that the slide member 42' performs a so-called "sliding" when the leg portions (31,32) extend from the first folded position to the second extended position as illustrated in Figs. 29, 35 and 38.
That is, the above slide member 42' may be any embodiment (shape) so long as it can perform a so-called "sliding". For example, it may be formed in the form of a "curved wall", etc., in place of the "slide wall 45'" that is formed in the form of being substantially "nearly horizontal" "nearly plate". In concert therewith, the connection hub 50H' of the fixing member 5' may be also formed in the form of a "curved wall" in place of the "inclined wall 60". The "curved wall" is also included in the "slide wall".

### [First latching portion 48, second latching portion 46 and third latching portion 47 of shield member]

The first latching portion 48, second latching portion 46 and third latching portion 47 of the shield member may have any constitution so long as they work to keep the connection hub 40H' of the shield member from being pulled out of the connection hub 50H' of the fixing member 5 as described above when the first and second leg portions (31,32) extend from the first folded position to the second extended position.
That is, the first latching portion 48 may have the form of a so-called "(hook) nail" or "a sword guard (flange)" in place of the form of "protrusion" (a nearly trapezoidal form) as illustrated in Fig. 38.
Further, the second latching portion 46 may have the form of a so-called "a sword guard (flange)" or the form of "protrusion" in place of a so-called "(hook) nail" as illustrated in Fig. 38.
Furthermore, the third latching portion 47 may have the form of a "(hook) nail" or the form of "protrusion" in place of a so-called "a sword guard (flange)" as illustrated in Fig. 38.

### [Connection hub 50H' of fixing member 5']

The connection hub 50H' of the fixing member 5' may have any constitution so long as it works to keep the connection hub 40H' of the shield member 3 from being pulled out as described above.
The first space 53 may have any constitution so long as it can contain the connection hub 40H' and fix the same therein.
The second space 59 may have any constitution so long as it can contain or accommodate the shield hub 35 of the shield member 3', the first and second leg portions (31,32) in the first folded position and the wings 34 therein (can have these thereon).
The first side wall 52, the intermediate wall 57 and the second side wall 63 may have any constitution so long as they can section the connection hub 50H' of the fixing member 5' into the first space 53 and the second space 59 as described above.
The cover 51' may have any constitution in which it works to fix the connection hub 40H' of the shield member 3' as described above.
The first latching portion 55' and second latching portion 56' of the fixing member may have any constitution so long as they can be latched and kept fixed by each other as described above.

### [Needle guide member 70]

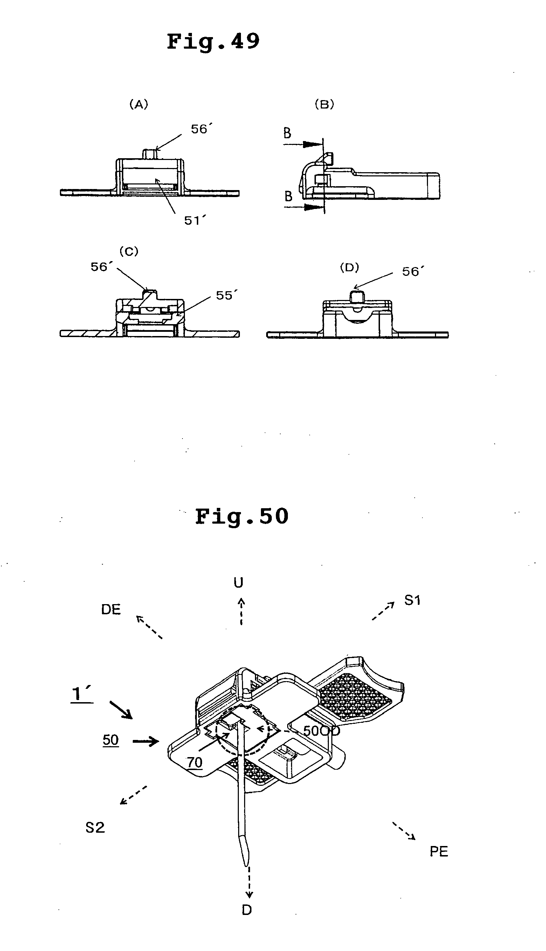

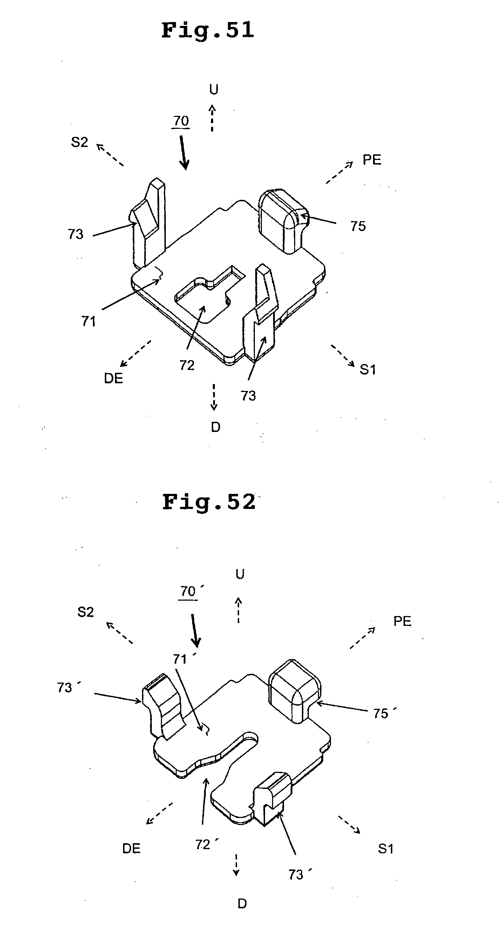

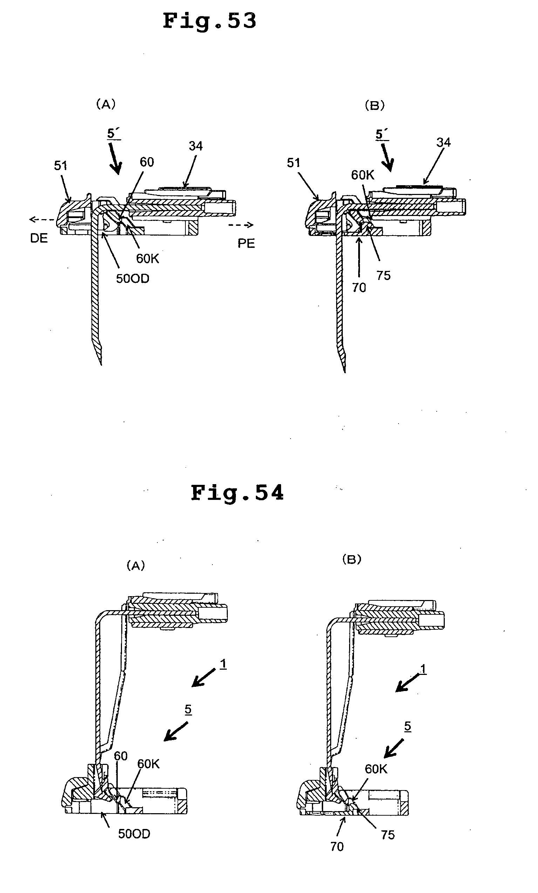

In the safety apparatus 1' for medical needles according to this invention (second invention (embodiment)), a needle guide member 70 for stably moving the needle upward can be attached to the lower opening portion 50OD of downward portion D of the fixing member 5' as illustrated in Fig. 50, (and more specifically in Fig.51.).
In the fixing member 5', the first side wall latching groove 52M is formed near to the distal end DE of the first side wall 52 [inside the first side portion S1 and inside the second side portion S2 thereof] for attaching the needle guide member 70 as illustrated in Figs. 41 and 47.
In the fixing member 5', further, the needle guide member latching portion 60K (to be also referred to as "latching groove") is formed somewhere in the middle of the slide wall 60 or near to the downward portion D as illustrated in Fig. 53(A).

The needle guide member 70 has a flat nearly plate-shaped wall 71 (to be simply abbreviated as "wall 71" hereinafter) as illustrated in Fig. 51.
In the wall 71, a needle passage hole 72 is formed from nearly a middle portion to the distal end DE side.
The needle passage hole 72 is formed in a nearly convex form combining two so-called "rectangular forms", and the distal end DE side thereof is larger than the nearly middle portion thereof.
In the wall 71, further, a needle guide member first latching portion 73 (to be simply abbreviated as "first latching portion 73" hereinafter) is formed on the first side portion S1 side and the second side portion S2 side.
The first latching portion 73 is arranged (formed) near to the distal end DE of the first side portion S1 side (second side portion S2 side) in a manner to rise up toward the upper portion U. When viewed from the first side portion S1 side (second side portion S2 side), the first latching portion 73 is formed in the form of a so-called "nearly the letter L".

In the wall 71, a needle guide member second latching portion 75 is formed on the proximal end PE side.
The second latching portion 75 is arranged (formed) nearly in the middle on the proximal end side in a way to rise up toward the upper portion U. When viewed from the first side portion S1 side (second side portion S2 side), the second latching portion 75 is formed in the form of "nearly the letter L".

The first latching portion 73 of the needle guide member 70 is latched and kept fixed in the first side wall latching groove 52M of the fixing member 5'.
The second latching portion 75 is, as shown in Fig.53, latched and kept fixed by the needle guide member latching portion 60K of slide wall 60 of the fixing member 5'.
The wall 71 covers a most part of lower opening portion 50OD of the fixing member 5' as illustrated in Figs. 50 and 53(B).
The needle 21 moves in the direction of the upper portion U through the needle passage hole 72 (having a far smaller opening area than the lower opening portion 50OD to which the wall 71 is not attached) formed in the wall 21.
Therefore, during the upward movement of the needle 21, its lateral wobbling is limited and constrained within the small needle passage hole 72, the needle can move in the direction of the upper portion U stably without much swinging during the movement.

### [Needle guide member 70']

The needle guide member 70' illustrated in Fig. 52 can be used in place of the needle guide member 70 shown in Fig. 51.
The needle guide member 70' differs from the needle guide member 70 in forms (shapes)of the needle passage hole 72' and the first latching portion 73'.
In the wall 71', the needle passage hole 72' is formed from a nearly middle portion to the distal end DE side (as an outwardly open shape).
The needle passage hole 72' is formed in a so-called "approximate snowman form" that shape is a combination of two so-called "approximate circle or ellipse forms". The distal end DE side is larger than the middle portion, and the distal end side is opened.
When viewed from the distal end DE side (proximal end PE side), the entirety of the first latching portion 73' is formed in the shape of "nearly the letter L".

The needle guide member 70' differs from the needle guide member 70 in forms (shapes) of the needle passage hole 72' and first latching portion 73' of the wall 71' as described above, while the first latching portion 73' can be latched and kept fixed in the first side wall latching portion 52M of the fixing member 5' like the needle guide member 70.
The second latching portion 75', like the second latching portion 75, can be latched and kept fixed in the needle guide member latching portion 60K of slide wall 60 of the fixing member 5'.
The wall 71' can cover a most part of lower opening portion 50OD of the fixing member 5'.
The needle 21 moves in the direction of the upper portion U through the needle passage hole 72' (having a far smaller opening area than the lower opening portion 50OD to which the wall 71' is not attached) formed in the wall 21. Therefore, during the upward movement of the needle 21, its lateral wobbling is confined within the needle passage hole 72', the needle can move in the direction of the upper portion U stably without much swinging during the movement.

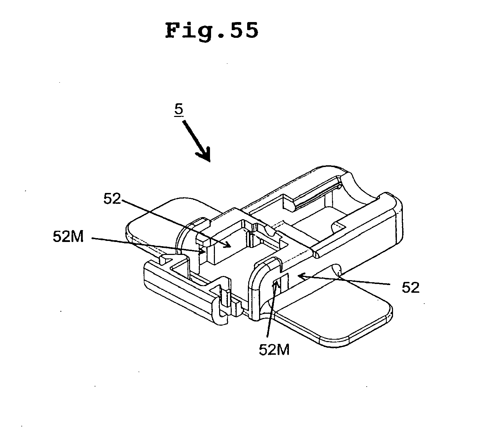

In the safety apparatus (embodiment) 1 for medical needles according to the first invention, further, the needle guide member (70, 70') can be attached thereto, and the needle 21 can be moved in the direction of the upper portion U stably without much lateral waggling backlash during the movement like the safety apparatus 1' for medical needles according to the second invention (embodiment).
That is, the above needle guide member (70, 70') can be attached to the lower opening portion 50OD of downward portion D of the fixing member 5 as illustrated in Figs. 54 and 55.
In the fixing member 5, the first side wall latching groove 52M is formed near to the distal end DE (inside of the first side portion S1 and inside of the second side portion S2) of the first side wall 52 for attaching the needle guide member (70,70').
In the fixing member 5, further, the needle guide member latching portion 60K (to be also referred to as "latching groove") is formed in a nearly middle portion of the slide wall 60 or near to the distal end DE side as illustrated in Fig. 54(A).

Since the shield member 3' and the fixing member 5' for constituting the safety apparatus 1' for medical needles can be formed from the same material by integral molding, they can be easily produced and can be produced at a low coast as compared with the apparatus described in Patent Document 1.
In the shield member 3', the shield hub 35, the leg portions (31, 32) and the connection hub 40H' are preferably formed from the same material by integral molding.
In the connection hub 40H', the second latching portion 46 and the third latching portion 47 can be formed from the same material by integral molding on each of the first side portion S1 side and the second side portion S2 side.
In the fixing member 5, the connection hub 50H' and the flat contact surface plate 50 are formed from the same material by integral molding.
In the connection hub 50H', preferably, a plurality of the walls (52, 57 and 63), the cover 51', the third latching portion 58 and the slide wall (inclined wall) 60 are formed from the same material by integral molding.
The material for the shield member 3' and the fixing member 5' is preferably a thermoplastic synthetic resin that can be used for injection molding, etc., like the first invention (embodiment). Examples of the material include PE (polyethylene), PP (polypropylene), PB (polybutylene), PS (polystyrene), PVC (polyvinyl chloride), PVdC (polyvinylidene chloride), PMMA (polymethyl methacrylate), PMA (polymethyl acrylate), PC (polycarbonate), PAm (Polyamide) and ETFE copolymers, while the material shall not be limited thereto like the first invention (embodiment).

The preferable embodiments of this invention are as follows.
[1] A safety apparatus (1) for medical needles, which includes a needle member (2), a shield member (3) and a fixing member (5), said needle member (2) having a needle (21) having a distal end (23), said needle (21) having a proximal end PE side fixed to a needle hub (22),
   said shield member (3) having a shield hub (35), a shield member connection hub (40H) and leg portions (31,32),
   said fixing member (5) having a fixing member connection hub (50H),
   the shield member connection hub (40H) of said shield member (3) having a slide member (42),
   said shield member connection hub (40H) being to form a space (40S) capable of containing the distal end (23) of said needle (21) and a wall (43DW) of said slide member (42),
   said shield member connection hub (40H) having an opening portion (400) through which the needle (21) can pass toward a downward portion D,
   said leg portions (31,32) being extendable from a first folded position to a second extended position,
   said wall (43DW) of the slide member (42) shifting together with said leg portions (31, 32) up to a position where it can block the cross section of said space (40S) when said leg portions (31,32) extends from a first folded position to a second extended position,
   said needle (21) being inserted into the space (40S) of said connection hub (40H) when said leg portions (31,32) are in the first folded position, the wall (43DW) of said slide member (42) being pulled into the space (40S) of said shield member connection hub (40H),
   whereby the wall (43DW) of said slide member (42) blocks the cross section of the space (40S) of said shield member connection hub (40H) to inhibit the distal end (23) of said needle from moving to a downward portion D lower than the wall (43DW) of said slide member (42), the apparatus being so formed as to prevent the distal end (23) of said needle (21) from being exposed to an outside.

[2] A safety apparatus (1) for medical needles as recited in the above [1], wherein the shield member connection hub (40H) of said shield member (3) has said slide member (42) formed on the proximal end PE side,
   has a shield member first latching portion (48) formed on the distal end DE side, and has a shield member second latching portion (46) and a shield member third latching portion (47) formed on each of a first side portion S1 side and a second side portion S2 side, and
   said shield member second latching portion (46) is formed in an upper portion U above said shield member third latching portion (47).

[3] A safety apparatus (1) for medical needles as recited in the above [1] or [2], wherein the fixing member connection hub (50H) of said fixing member (5) has a plurality of walls (52,57,63) and a cover (51),
   the space of said fixing member connection hub (50H) is sectioned into a first space (53) and a second space (59)
   by said plurality of walls (52,57,63) and a wall (51W) of said cover (51),
   and said first space (53) can contain the shield member connection hug (40H) of said shield member (3).

[4] A safety apparatus (1) for medical needles as recited in any one of the above [1] to [3], wherein the plurality of walls (52,57,63) of fixing member connection hub (50H) of said fixing member (5) has
   a pair of first side walls (52) extending in the length direction of a horizontal axial line HL,
   a second side wall (63) formed in the direction from the first side portion S1 to the second side portion S2 on the proximal end PE side of the fixing member connection hub (50H),
   an intermediate wall (57) formed somewhere on said pair of first side walls (52) in the direction from the first side portion S1 to the second side portion S2, and
   a slide wall (60) formed on the proximal end side of said intermediate wall (57), and
   a third latching portion (58) is formed on the distal end side of said intermediate wall (57).

[5] A safety apparatus (1) for medical needles as recited in any one of the above [1] to [4], wherein the slide member (42) of shield member connection hub (40H) of said shield member (3) has
   a distal end side wall (43DW) and a downward portion wall (42B),
   a slide wall (45) is formed on the first side S1 side and the second side portion S2 side, and
   a groove (43) is formed in said slide wall (45).

[6] A safety apparatus (1) for medical needles as recited in the above [4] or [5], wherein, when the shield member connection hub (40H) of said shield member (3) and the connection hub (50H) of said fixing member (5) are connected to each other,
   the fixing member third latching portion (58) of fixing member connection hub (50H) of said fixing member (5) is latched and kept immobile by the groove (43) of slide member (43) of said shield member (3), and
   the slide wall (45) of said slide member (42) is brought into contact with the slide wall (60) of said intermediate wall (57), and
   when said leg portions (31,32) extend from the first folded position to the second extended position, the slide member (42) of the said shield member connection hub (40H) is therewith pulled into the space (40S) of said connection hub (40H), and
   the distal end side wall (43DW) of said slide member (42) shifts up to a position where it can block the cross section of said space (40S).

[7] A safety apparatus (1) for medical needles as recited in any one of the above [1] to [6], wherein, when said leg portions (31,32) are in the first folded position,
   the second latching portion (46) of shield member connection hub (40H) of said shield member (3) is in a position of downward portion of first upper wall (61) of fixing member connection hub (50H) of said fixing member (5),
   and when said leg portions (31,32) extend from the first folded position to the second extended position,
   the shield member second latching portion (46) of said shield member connection hub (40H) is latched and kept immobile by an upper portion of the first upper wall (61), and the shield member third latching portion (47) of said shield member connection hub (40) is latched and kept immobile by a downward portion of the first upper wall (61),
   so that said shield member connection hub (40H) can be prevented from coming off from the fixing member connection hub (50H) of said fixing member (5).

[8] A safety apparatus (1) for medical needles as recited in any one of the above [4] to [7], wherein a groove (43) is formed in the slide wall (45) of shield member connection hub (40H) of said shield member (3) and the fixing member third latching portion (58) of intermediate wall (57) of fixing member connection hub (50H) of said fixing member (5) is formed in the form of a rib for latching and keeping it immobile in said groove (43), or
   wherein a latching portion in the form of a rib is formed in the slide wall (45) of shield member connection hub (40H) of said shield member (3), and a groove is formed in intermediate wall (57) of fixing member connection hub (50H) of said fixing member (5) for lathing said latching portion in the form of a rib and keeping it immobile.

[9] A safety apparatus (1) for medical needles as recited in any one of the above [4] to [8], said shield hub (35), sad leg portions (31,32) and said shield member connection hub (40H) of said shield member (3) are an integrated product formed from the same material by integral molding.

[10] A safety apparatus (1) for medical needles as recited in any one of the above [4] to [9], wherein said fixing member (5) has a needle guide member (70,70'), and said needle guide member (70,70') is attached to a lower opening portion (50OD).

[11] A safety apparatus (1) for medical needles as recited in any one of the above [4] to [10], wherein said needle guide member (70,70') has a nearly plate-shaped wall (71,71'), said wall (70,70') has a needle passage hole (72,72'), and said wall (71,71') has a needle guide member first latching portion (73,73') and a needle guide member second latching portion (75,75').

[12] A safety apparatus (1) for medical needles as recited in any one of the above [4] to [11], wherein:
   said fixing member (5) has a first side wall latching groove (52M) in the first side wall (52),
   said fixing member (5) has a needle guide member latching portion (60K) somewhere in the middle of the slide wall (60) or on the downward portion D side thereof,
   the needle guide member first latching portion (73,73') of said needle guide member (70,70') is lathed and kept immobile in said first side wall latching groove (52M), and
   the needle guide member second latching portion (75,75') of said needle guide member (70,70') is latched and kept immobile in said needle guide member latching portion (60K).

[13] A safety apparatus (1) for medical needles as recited in any one of the above [4] to [12], wherein:
   said needle (21) is bent toward the vertical axial line VL side at a predetermined angle with regard to the horizontal axial line HL somewhere in its length from the proximal end side to the distal end DE side, and
   the needle hub (22) on the proximal end PE side of said needle (21) is fixed to the said shield hub (35).

[14] A safety (1') apparatus for medical needles, which includes a needle member (2), a shield member (3') and a fixing member (5'),
   said needle member (2) having a needle (21) having a distal end (23), said needle (21) having a proximal end side fixed to a needle hub (22),
   said shield member (3') having a shield hub (35), a shield member connection hub (40H') and leg portions (31,32),
   said fixing member (5') having fixing member connection hub (50H'),
   the shield member connection hub (40H') of said shield member (3') having a slide member (42'),
   said shield member connection hub (40H') having a space (40S) capable of containing the distal end (23) of said needle (21) and walls (42B',45') of said slide member (42'),
   said shield member connection hub (40H') having an opening portion (40O') through which the needle (21) can pass in the downward portion D,
   said leg portions (31,32) being capable of extending from the first folded position to the second extended position,
   the walls (42B',45') of said slide member (42) shifting together with said leg portions (31,32) up to a position where they can block cross section of the opening portion (40O') of said connection hub (40H') when said leg portions (31,32) extend from the first folded position to the second extended position,
   said needle (21) being inserted into the space (40S) of said shield member connection hub (40H') when said leg portions (31,32) are in the first folded position,
   the wall (42B') of said slide member (43') being pulled into the space (40S) of said shield member connection hub (40H') when said leg portions (31,32) extend to the second extended position,
   whereby the wall (42B') of said slide member (42') being to block the cross section of the opening portion (40O') of said shield member connection hub (40H') to inhibit the distal end (23) of said needle (21) from moving to the downward portion D lower than the wall (42B') of said slide member (42'),
   the apparatus being so formed that it can prevent the distal end (23) of said needle (21) from being exposed to an outside.

[15] A safety apparatus (1') for medical needles as recited in the above [1], wherein the shield member connection hub (40H') of said shield member (3) has said slide member (42') formed on the proximal end PE side,
   has a shield member first latching portion (48) formed on the distal end side and a shield member second latching portion (46) and a shield member third latching portion (47) formed on each of the first side portion S1 side and the second side portion S2 side, and
   said shield member second latching portion (46) is formed in an upper portion U above said shield member third latching portion (46).

[16] A safety apparatus (1') for medical needles as recited in the above [14] or [15], wherein the fixing member connection hub (50H') of said fixing member (5) has a plurality of walls (52,57,63) and a cover (51'),
   the space of said fixing member connection hub (50H') is sectioned into a first space (3) and a second space (59)
   by said plurality of walls (52,57,63) and a wall (51W) of said cover (51'), and
   said first space (53) can contain the shield member connection hub (40H') of said shield member (3').

[17] A safety apparatus (1') for medical needles as recited in any one of the above [14] to [16], wherein the plurality of walls (52,57,63) of fixing member connection hub (50H') of said fixing member (5) include
   a pair of first side walls (52) extending in the length direction of a horizontal axial line HL,
   a second side wall (63) formed in the direction from the first side portion S1 to the second side portion S2 on the proximal end PE side of the fixing member connection hub (50H'),
   an intermediate wall (57) formed somewhere on said pair of first side walls (52) in the direction from the first side portion S1 to the second side portion S2, and
   a slide wall (60) formed on the proximal end side of said intermediate wall (57).

[18] A safety apparatus (1') for medical needles as recited in any one of the above [14] to [17], wherein the slide member (42') of shield member connection hub (40H') of said shield member (3') has a slide wall (45') and a downward portion wall (42B').

[19] A safety apparatus (1') for medical needles as recited in any one of the above [14] or [15], wherein, when the shield member connection hub (40H') of said shield member (3') and the fixing member connection hub (50H') of said fixing member (5') are connected to each other,
   the slide wall (45') of said slide member (42') is brought into contact with the slide wall (60) of said intermediate wall (57), and
   when said leg portions (31,32) extend from the first folded position to the second extended position, the slide member (42') of said shield member connection hub (40H') is cooperatively therewith pulled into the space (40S') of said shield member connection hub (40H'), and
   the downward portion wall (42B') of said slide member (42') can shift its position up to a position where it can block the cross section of said opening portion (40O').

[20] A safety apparatus (1') for medical needles as recited in any one of the above [14] to [19], wherein, when said leg portions (31,32) are in the first folded position,
   the shield member second latching portion (46) of shield member connection hub (40H') of said shield member (3') is in a position of downward portion of first upper wall (61) of fixing member connection hub (50H') of said fixing member (5), and
   when said leg portions (31,32) extend from the first folded position to the second extended position,
   the shield member second latching portion (46) of said shield member connection hub (40H') is latched and kept immobile by an upper portion of the first upper wall (61), and the shield member third latching portion (47) is latched and kept immobile by a downward portion of the first upper wall (61),
   whereby said shield member connection hub (40H') can be prevented from being pulled out of the fixing member connection hub (50H') of said fixing member (5').

[21] A safety apparatus (1') for medical needles as recited in any one of the above [14] to [20], wherein said cover (51') has a novel fixing member second latching portion (56') in an upper portion U, and
   when the shield member connection hub (40H') of said shield member (3') and the fixing member connection hub (50H') of said fixing member (5') are connected to each other,
   said novel fixing member second latching portion (56') presses the shield member connection hub (40H') of said shield member (3') from the direction of an upper portion U to keep the cover (51') that is once closed from opening.

[22] A safety apparatus (1') for medical needles as recited in any one of the above [14] to [21], wherein said shield hub (35), said leg portions (31,32) and said shield member connection hub (40H') of said shield member (3') are an integrated product formed from the same material by integral molding.

[23] A safety apparatus (1') for medical needles as recited in any one of the above [14] to [22], wherein said fixing member (5') has a needle guide member (70,70'), and said needle guide member (70,70') is attached to a lower opening portion (50OD).

[24] A safety apparatus (1') for medical needles as recited in any one of the above [14] to [23], wherein said needle guide member (70,70') has a nearly plate-shaped wall (71,71'),
   said wall (71,71') has a needle passage hole (72,72'), and
   said wall (71,71') has a needle guide member first latching portion (73,73') and a needle guide member second latching portion (75,75').

[25] A safety apparatus (1') for medical needles as recited in any one of the above [14] to [24], wherein said fixing member (5') has a first side wall latching groove (52M) in the first side wall (52),
   said fixing member (5') has a needle guide member latching portion (60K) on the downward portion D side of the slide wall (60),
   the needle guide member first latching portion (73,73') of said needle guide member (70,70') is latched and kept immobile in said first side wall lathing groove (52M), and
   the needle guide member second latching portion (75,75') of said needle guide member (70,70') is latched and kept immobile in said needle guide member latching portion (60K).

[26] A safety apparatus (1') for medical needles as recited in any one of the above [14] to [25], wherein said needle (21) is bent toward the vertical axial line VL side at a predetermined angle with regard to the horizontal axial line HL somewhere in its length from the proximal end PE side to the distal end DE side, and
   the needle hub (22) on the proximal end PE side of said needle (21) is fixed to said shield hub (35).
   Hereinafter, the first invention (embodiment) and the second invention (embodiment) will be generally referred to as "this invention", and when distinguished, these will be explained as "the first invention (embodiment)" and "the second invention (embodiment)".

### [Industrial Utility]

This invention has the following industrial utility.
The safety apparatus for medical needles according to this invention can reliably prevent the distal end of a used needle from being exposed to an outside. Therefore, the needle distal end having the blood, etc., of a patient adhering thereto, contained in the apparatus, is in no case accidentally exposed to an outside, so that workers can dispose of it with a sense of security. Further, even if the needle distal end has the blood, etc., of a patient adhering thereto, hygienically, the apparatus can prevent them from sputtering of stained blood.
According to the safety apparatus for medical needles according to this invention, the shield hub constituting the shield member, the leg portions and the shield hub connection hub can be formed from the same material by integral molding, so that the apparatus can be easily and inexpensively produced.

### [Explanation of symbols]

1,1': Safety apparatus for medical needles
2: Needle member
3,3': Shield member
5,5': Fixing member
6: Needle cover
HL: Horizontal axial line
VL: Vertical axial line
PE: Proximal end
DE: Distal end
S1: First side portion
S2: Second side portion
U: Upper portion
D: Downward portion
21: Needle
22: Needle hub
23: Distal end
31: First leg portion
32: Second leg portion
34: Wing
34H: Hinge
35: Shield hub
35S: Cylindrical space portion
37: Proximal end side hinge
38: Distal end side hinge
39: Intermediate hinge
40H, 40H': Shield member connection hub
40S: Space
400, 400': Opening portion
41: Upper portion connection portion
42, 42': First slide member, Second Slide member
42B, 42B': Downward portion wall
42BS': Cut portion
43DW: Wall (distal end side wall)
43: Groove
44: Hinge
45, 45': Slide wall (inclined wall)
46: Shield member second latching portion
47: Shield member third latching portion
48: Shield member first latching portion
50: Flat contact surface plate
50OU: Upper opening portion
50OD: Lower opening portion
50H: Fixing member connection hub
51, 51': Cover
51W: Cover wall
52: First side wall
52M: First side wall latching groove
53: First space
54: Hinge
55, 55': Fixing member first latching portion
56, 56': Fixing member second latching portion (pressing member)
57: Intermediate wall
58: Fixing member third latching portion
59: Second space
60: Slide wall (inclined wall)
60K: Needle guide member latching portion
61: First upper wall
62: Second upper wall
63: Second side wall
63C: Curved surface
T: Tube
70, 70': Needle guide member
71, 71': Wall
72, 72': Needle passage hole
73, 73': Needle guide member first latching portion
75, 75': Needle guide member second latching portion

## Claims

1. A safety apparatus (1,1') for medical needles, which includes a needle member (2), a shield member (3,3') and a fixing member (5,5'), said needle member (2) having a needle (21) having a distal end (23), said needle (21) is fixed to a needle hub (22) on a proximal end PE side,
said shield member (3,3') having a shield hub (35), a shield member connection hub (40H,40H') and leg portions (31,32),
said fixing member (5,5') having a fixing member connection hub (50H,50H'),
the shield member connection hub (40H,40H') of said shield member (3,B) having a first slide member (42) or a second slide member (42'),
said shield member connection hub (40H,40H') having formed a space (40S) capable of containing or accommodating the distal end (23) of said needle (21) and a wall (43DW) of said first slide member (42), or walls (42B',45') of said second slide member (42')
said shield member connection hub (40H,40H') having an opening portion (400, 40O') through which the needle (21) can pass toward a downward portion D,
said leg portions (31,32) being extendable from a first folded position to a second extended position,
said wall (43DW) of the first slide member (42) shifting together with said leg portions (31, 32) up to a position where it can block the cross section of said space (40S) when said leg portions (31,32) extends from a first folded position to a second extended position, or
said walls (42B',45') of said second slide member (42') shifting together with said leg portions (31,32) up to a position where they can block the cross section of the opening portion (40O' of said connection hub (40H') when said leg portions (31,32) extend from the first folded position to the second extended position,
said needle (21) being inserted into the space (40S) of said connection hub (40H, 40H') when said leg portions (31,32) are in the first folded position, the wall (43DW) of said first slide member (42) or said walls (42B',45') of said second slide member (42') being pulled into the space (40S) of said shield member connection hub (40H, 40H'), when said leg portions (31,32) are in the second extended position,
whereby the wall (43DW) of said first slide member (42) blocks the cross section of the space (40S) of said shield member connection hub (40H) to prevent the distal end (23) of said needle from moving to a downward portion D lower than the wall (43DW) of said slide member (42), and or
whereby the wall (42B') of said second slide member (42') blocks the cross section of the opening portion (40O') of said shield member connection hub (40H') to prevent the distal end (23) of said needle (21) from moving to the downward portion D lower than the wall (42B') of said slide member (42'), and
the apparatus being so formed as to prevent the distal end (23) of said needle (21) from being exposed to an outside.

2. The safety apparatus (1,1') for medical needles as recited in claim 1, wherein the shield member connection hub (40H, 40H') of said shield member (3,3') has said first slide member (42), or said second slide member (42'),each formed on the proximal end PE side,
has a shield member first latching portion (48) formed on the distal end DE side, and has a shield member second latching portion (46) and a shield member third latching portion (47) formed on each of a first side portion S1 side and a second side portion S2 side, and
said shield member second latching portion (46) is formed in an upper portion U above said shield member third latching portion (47).

3. The safety apparatus (1,1') for medical needles as recited in claim 1 or 2, wherein the fixing member connection hub (50H,50H') of said fixing member (5,5') has a plurality of walls (52,57,63) and a cover (51,51') having a wall (51W),
the space of said fixing member connection hub (50H,50H') is sectioned into a first space (53) and a second space (59),
by said plurality of walls (52,57,63) and said wall (51W) of said cover (51,51'),
and said first space (53) can contain or accommodate the shield member connection hub (40H, 40H') of said shield member (3,3').

4. The safety apparatus(1,1') for medical needles as recited in any one of claims 1 to 3, wherein the plurality of walls (52,57,63) of the fixing member connection hub (50H,50H') of said fixing member (5,5') has,
a pair of first side walls (52) extending in the longitudinal direction of a horizontal axial line HL,
a second side wall (63) formed in the direction from the first side portion S1 to the second side portion S2 on the proximal end PE side of the fixing member connection hub (50H),
an intermediate wall (57) formed somewhere on said pair of first side walls (52) in the direction from the first side portion S1 to the second side portion S2, and
a slide wall (60) formed on the proximal end side of said intermediate wall (57).

5. The safety apparatus (1) for medical needles as recited in any one of claims 1 to 4, wherein the plurality of walls (52,57,63) of fixing member connection hub (50H) of said fixing member (5) has,
a pair of first side walls (52) extending in the longitudinal direction of a horizontal axial line HL,
a second side wall (63) formed in the direction from the first side portion S1 to the second side portion S2 on the proximal end PE side of the fixing member connection hub (50H),
an intermediate wall (57) formed somewhere on said pair of first side walls (52) in the direction from the first side portion S1 to the second side portion S2, and
a slide wall (60) formed on the proximal end side of said intermediate wall (57), and
a third latching portion (58) is formed on the distal end side of said intermediate wall (57),and
when the shield member connection hub (40H) of said shield member (3) and the connection hub (50H) of said fixing member (5) are connected to each other,
the fixing member third latching portion (58) of fixing member connection hub (50H) of said fixing member (5) is latched and kept fixed by the groove (43) of first slide member (42) of said shield member (3), and
the slide wall (45) of said first slide member (42) is brought into contact with the slide wall (60) of said intermediate wall (57), and
when said leg portions (31,32) extend from the first folded position to the second extended position, the first slide member (42) of the said shield member connection hub (40H) is therewith pulled into the space (40S) of said connection hub (40H), and
the distal end side wall (43DW) of said first slide member (42) shifts up to a position where it can block the cross section of said space (40S).

6. The safety apparatus (1) for medical needles as recited in any one of claims 1 to 5, wherein the first slide member (42) of shield member connection hub (40H) of said shield member (3) has
a distal end side wall (43DW) and a downward portion wall (42B),
a slide wall (45) is formed on the first side portion S1 side and the second side portion S2 side, and
a groove (43) is formed in said slide wall (45).

7. The safety apparatus (1,1') for medical needles as recited in any one of claims 1 to 6, wherein, when said leg portions (31,32) are in the first folded position,
the second latching portion (46) of shield member connection hub (40H, 40H') of said shield member (3,3') is in a downward position of first upper wall (61) of fixing member connection hub (50H,50H') of said fixing member (5,5'),
and when said leg portions (31,32) extend from the first folded position to the second extended position,
the shield member second latching portion (46) of said shield member connection hub (40H, 40H') is latched and kept fixed by an upper portion of the first upper wall (61), and the shield member third latching portion (47) of said shield member connection hub (40H, 40H') is latched and kept fixed by a downward portion of the first upper wall (61),
so that said shield member connection hub (40H, 40H') can be prevented from coming off from the fixing member connection hub (50H,50H') of said fixing member (5,5').

8. The safety apparatus (1) for medical needles as recited in any one of claims 4 to 7, wherein a groove (43) is formed in the slide wall (45) of shield member connection hub (40H) of said shield member (3) and the fixing member third latching portion (58) of intermediate wall (57) of fixing member connection hub (50H) of said fixing member (5) is formed in the form of a rib for intrudingly latching and keeping it fixed in said groove (43), or
wherein a latching portion in the form of a rib is formed in the slide wall (45) of shield member connection hub (40H) of said shield member (3), and a groove is formed in intermediate wall (57) of fixing member connection hub (50H) of said fixing member (5) for lathing said latching portion in the form of a rib and keeping it fixed.

9. The safety apparatus (1') for medical needles as recited in any one of claims 1 to 8, wherein the second slide member (42') of shield member connection hub (40H') of said shield member (3') has a slide wall (45') and a downward portion wall (42B'), and
wherein, when the shield member connection hub (40H') of said shield member (3') and the fixing member connection hub (50H') of said fixing member (5') are connected to each other,
the slide wall (45') of said second slide member (42') is brought into contact with the slide wall (60) of said intermediate wall (57), and
when said leg portions (31,32) extend from the first folded position to the second extended position, the second slide member (42') of said shield member connection hub (40H') is cooperatively therewith pulled into the space (40S') of said shield member connection hub (40H'), and
the downward portion wall (42B') of said second slide member (42') can shift up to a position where it can block the cross section of said opening portion (40O').

10. The safety apparatus (1') for medical needles as recited in any one of claims 3 to 9, wherein said cover (51') has a novel fixing member second latching portion (56') in an upper portion U, and
when the shield member connection hub (40H') of said shield member (3') and the fixing member connection hub (50H') of said fixing member (5') are connected to each other,
said novel fixing member second latching portion (56') presses the shield member connection hub (40H') of said shield member (3') from the direction of an upper portion U to keep the cover (51') that is once closed from opening again.

11. The safety apparatus (1,1') for medical needles as recited in any one of claims 1 to 10, wherein said shield hub (35), said leg portions (31,32) and said shield member connection hub (40H,40H') of said shield member (3,3') are an integrated product formed from the same material by integral molding.

12. The safety apparatus (1,1') for medical needles as recited in any one of claims 1 to 11, wherein said fixing member (5,5') has a needle guide member (70,70'), and said needle guide member (70,70') is attached to a lower opening portion (50OD).

13. The safety apparatus (1,1') for medical needles as recited in claim 12, wherein said needle guide member (70,70') has a nearly plate-shaped wall (71,71'),
said wall (71,71') has a needle passage hole (72,72'), and
said wall (71,71') has a needle guide member first latching portion (73,73') and a needle guide member second latching portion (75,75').

14. The safety apparatus (1,1') for medical needles as recited in claim 12 or 13, wherein said fixing member (5,5') has a first side wall latching groove (52M) in the first side wall (52),
said fixing member (5,5') has a needle guide member latching portion (60K) on the downward portion D side of the slide wall (60),
the needle guide member first latching portion (73,73') of said needle guide member (70,70') is latched and kept fixed in said first side wall lathing groove (52M), and
the needle guide member second latching portion (75,75') of said needle guide member (70,70') is latched and kept fixed in said needle guide member latching portion (60K).

15. The safety apparatus (1,1') for medical needles as recited in any one of claims 1 to 14, wherein said needle (21) is bent toward the vertical axial line VL side at a predetermined angle with regard to the horizontal axial line HL somewhere in its length from the proximal end PE side to the distal end DE side, and
the needle hub (22) on the proximal end PE side of said needle (21) is fixed to said shield hub (35).
